(19) Europäisches Patentamt / European Patent Office / Office européen des brevets

(11) **EP 4 408 572 B1**

(12) **EUROPEAN PATENT SPECIFICATION**

(45) Date of publication and mention
of the grant of the patent:
**01.07.2026 Bulletin 2026/27**

(21) Application number: **22783421.5**

(22) Date of filing: **23.09.2022**

(51) International Patent Classification (IPC):
**B01D 61/14** (2006.01)     **B01D 61/16** (2006.01)
**B01D 15/02** (2006.01)     **B01D 15/24** (2006.01)
**B01D 65/02** (2006.01)     **B01J 20/28** (2006.01)
**C07K 1/16** (2006.01)      **C07K 1/34** (2006.01)
**C07K 1/36** (2006.01)

(52) Cooperative Patent Classification (CPC):
(C-Sets available)
**B01D 61/145; B01D 15/02; B01D 15/24;**
**B01D 61/147; B01D 61/16; B01D 65/02;**
**C07K 1/34;** B01D 2311/04; B01D 2311/08;
B01D 2311/25; B01D 2311/2626; B01D 2321/04
(Cont.)

(86) International application number:
**PCT/DK2022/050195**

(87) International publication number:
**WO 2023/051885 (06.04.2023 Gazette 2023/14)**

(54) **IMPROVED PROTEIN SORPTION AND FILTRATION APPARATUS**

VORRICHTUNG ZUR SORPTION UND FILTRATION VON PROTEINEN

APPAREIL AMÉLIORÉ DE SORPTION ET DE FILTRATION DE PROTÉINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2021 DK PA202170471**

(43) Date of publication of application:
**07.08.2024 Bulletin 2024/32**

(73) Proprietor: **LPS Biosolutions A/S**
**2750 Ballerup (DK)**

(72) Inventors:
• **LIHME, Allan Otto Fog**
**2750 Ballerup (DK)**
• **HANSEN, Marie Bendix**
**2750 Ballerup (DK)**
• **LINDVED, Bodil Kjær**
**2750 Ballerup (DK)**
• **LIHME, Peter Fog**
**2750 Ballerup (DK)**
• **HANSEN, Dennis Kim**
**2750 Ballerup (DK)**
• **STIGSBY, Oliver Benjamin**
**2750 Ballerup (DK)**

(74) Representative: **Plougmann Vingtoft a/s**
**Strandvejen 70**
**2900 Hellerup (DK)**

(56) References cited:
WO-A1-2019/215153     WO-A1-2021/099529
US-A- 6 022 477       US-A1- 2002 170 859
US-A1- 2007 299 251   US-A1- 2014 284 274
US-A1- 2015 299 275   US-A1- 2019 300 396

• HARRIS E L V ET AL: "Protein purification methods a practical approach: Concentration of the extract", 1 January 1989, PROTEIN PURIFICATION METHODS : A PRACTICAL APPROACH; [THE PRACTICAL APPROACH SERIES], OXFORD [U.A.] : IRL PRESS, PAGE(S) 125 - 175, ISBN: 978-0-19-963002-8, XP002516106

- **MARCEL MULDER: "Basic Principles of Membrane Technology", 1 January 1991, 19910101, XP002571341**

(52) Cooperative Patent Classification (CPC): (Cont.)

C-Sets
B01D 2311/04, B01D 2311/2626;
B01D 2311/08, B01D 2311/25, B01D 2311/04

**Description**

**Technical field of the invention**

The present invention relates to separation of proteins or other biomolecules from compositions. In particular, the present invention relates to an apparatus for collective sorption and filtration of protein or other biomolecules.

**Background of the invention**

A major drawback for the filtration devices based on ultrafiltration and even microfiltration membranes having sub-micron porosities is the strong tendency to fouling with soluble and/or insoluble substances such as polyphenols, polysaccharides, lipids, protein aggregates and colloidal fibres. Such fouling is often uncontrollable and leads to a substantial loss of liquid flux through the filters. Many filtration devices are therefore equipped with systems to create a backward flush whereby the fouling material may be removed from the filter. The efficiency of such backward flush systems is, however, highly variable and dependent on the specific nature of the fouling mechanisms. Thus, in many cases it may be without significant effect to perform backward flushes, especially in cases involving fouling of sub-micron pores of membranes by substances adsorbed to the membrane surface structure (as opposed to physical entrapment and blockage with particles able to penetrate the pores). Fouling by adsorption may happen even with substances being fully or partly soluble in the liquid passing the filtration device, and the fouling substances may build up as multilayered structures coating the outer and inner surfaces of the filtration membrane. For membranes having sub-micron pore structures such adsorption based fouling may very quickly develop to such a degree that the pores gets blocked and liquid flux through the membrane is declining.

As such, WO2015/118223 A1 discloses an apparatus for purifying liquids containing impurities such as oils or dissolved metals. The filter in the apparatus is purified in dedicated purifying cycles employing a combination of high-power backwashing with ultra-sonication of the filter element.

Furthermore, US 6,022,477 A discloses an apparatus and method for recovering bioproducts from a feed solution. The apparatus includes a housing, a membrane disposed in the housing for filtering the bioproducts and an absorbent bed disposed in the housing for retaining the bioproducts which permeate through a membrane. The apparatus is adapted to allow fractionation and purification of the retained bioproducts from the bed by elution.

Filtration of complex mixtures, such as refining of biological compositions comprising proteins, is an application area often troubled by extensive fouling of the filter elements resulting in a substantial loss of liquid flux. An effective system for cleaning the filter is therefore considered a requirement when working in this field. However, many filter elements are not suitable for use with an efficient back flush cleaning system. This is for example the case for commonly used ultra- and microfiltration flat sheet membranes composed of a separating membrane layer (the skin layer) cast on top of a supporting membrane structure. Also, in a process for separation of proteins by collective sorption and filtration of a protein composition, the concentration of adsorbent in the composition may by very high. However, this in turn may lead to fast blocking of the filter surface by the adsorbent particles and a subsequent decline of liquid flux and thereby a lower process efficiency. Also, certain filter types are very prone to physical blockage of the tangential flow fluid path during operation at high adsorbent concentrations.

Hence, an improved apparatus for collective sorption and filtration of a biological composition would be advantageous, and in particular a more efficient and/or reliable apparatus for refining a protein or other biomolecule from a biological composition would be advantageous.

**Summary of the invention**

Thus, an object of the present invention relates to refining of proteins or other biomolecules from biological compositions by employing a collective sorption and tangential filtration apparatus with elution of the target protein or other biomolecule.

In particular, it is an object of the present invention to provide an apparatus that solves the above mentioned problems of the prior art with fouling of the filter surface, blockage of tangential flow fluid paths and low liquid flux leading to poor process efficiency.

Thus, a first aspect of the present invention relates to an apparatus comprising a first inlet 1 and one or more first fluid connections 3 configured to lead fluid from the first inlet 1 to a first end 4 of a tangential flow filtration unit 5 comprising a filter 6 with a nominal pore size in the range of 20 to 300 $\mu$m, such that at least 60% of particles larger than a value within 20 to 300 $\mu$m are retained by the filter having an average pore size of that value, and wherein the first fluid connections 3 comprises a sorption material for protein or other biomolecule suspended in an aqueous liquid at a concentration of 5-70 % by volume, and where a second end 7 of the tangential flow filtration unit 5 has a second fluid connection 8 configured to lead retentate back to the first end 4 of the tangential flow filtration unit 5; the tangential flow filtration unit 5 having a third fluid connection 9 configured to lead permeate to a first outlet 10; and wherein the apparatus comprises an automatic backward flush system being configured to apply a pulsed pressure increase and/or backward flush of the permeate with a frequency in the range of 1-500 per hour and/or a pulse duration of between 0.01 to 200 seconds.

A biomolecule, for example a protein, in a biological composition may be refined by a process e.g. utilizing the apparatus as described herein.

Thus, a second aspect of the present invention relates to a process for separation of a protein or other biomolecule comprised in a biological composition, the process comprising the steps of:

i) mixing a biological composition comprising a target protein or other biomolecule with a composition comprising a sorption material for protein or other biomolecule to obtain a mixture, wherein the target protein or other biomolecule is carried by the sorption material,

ii) filtrating the mixture using a tangential flow filtration unit having a membrane with nominal pore sizes in the range of 20 to 300 $\mu$m, such that at least 60% of particles larger than a value within 20 to 300 $\mu$m are retained by the filter having an average pore size of that value, for obtaining a first retentate comprising the target protein or other biomolecule and a first permeate which is optionally collected in a waste tank or a permeate collection tank,

iii) recirculating and filtrating the first retentate by returning the obtained first retentate to the tangential flow filtration unit and thereby obtain a second permeate which is optionally collected, and obtaining a second retentate, and wherein the recirculation and further filtration in step iii) is carried out one or more times,

iv) adding an elution liquid to the second retentate to release the target protein or other biomolecule from the sorption material,

v) filtrating the second retentate using the tangential flow filtration unit to obtain a third retentate comprising the sorption material and a third permeate comprising the target protein or another biomolecule;

vi) collecting the third permeate as a separated product composition comprising protein or other biomolecule, and wherein a pressure difference between the retentate side and permeate side of the filter in the tangential flow filtration unit is equalized or reversed resulting in a pulsed pressure increase and/or backward flush of the collected first permeate is obtained, said pressure difference is introduced at least once during the process during one or more of the steps ii) to iv), and wherein the pulsed pressure increase and/or backward flush is performed with a frequency in the range of 1-500 per hour and/or a pulse duration in the range of 0.01 to 200 seconds.

## Brief description of the figures

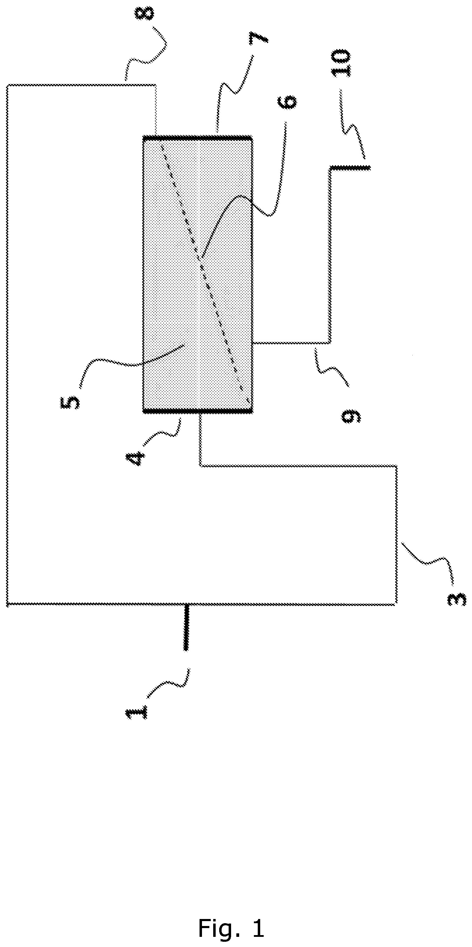

**Figure 1** shows, a schematic representation of an apparatus according to the present invention (backward flush system not shown), which comprises, a first inlet 1 being in fluid connection with first end 4 of a tangential flow filtration unit 5 through a first fluid connection 3. From a second end 7, a second fluid connection 8 is configured to lead the retentate back to the mixing tank 2, whereas a third fluid connection 9 is configured to lead the permeate to a first outlet 10.

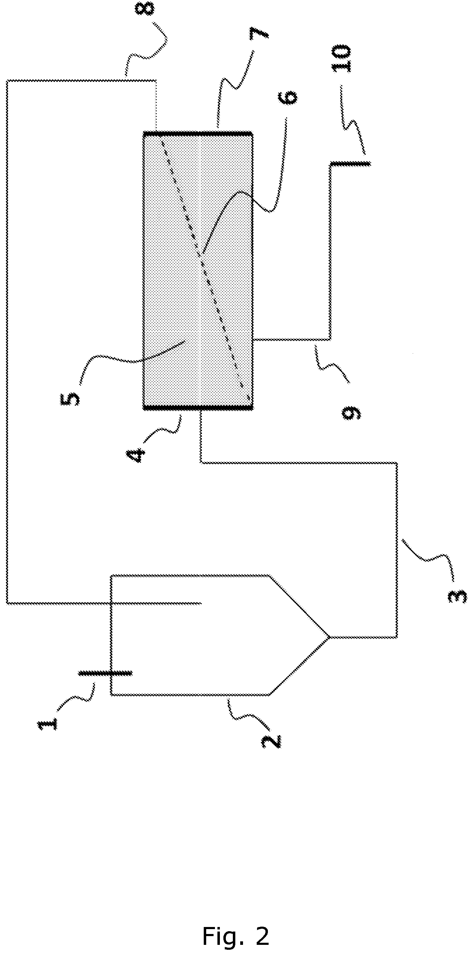

**Figure 2** shows, similarly to figure 1, a schematic representation of an apparatus according to the present invention (backward flush system not shown), but in figure 2 the embodiment of having a mixing tank is included.

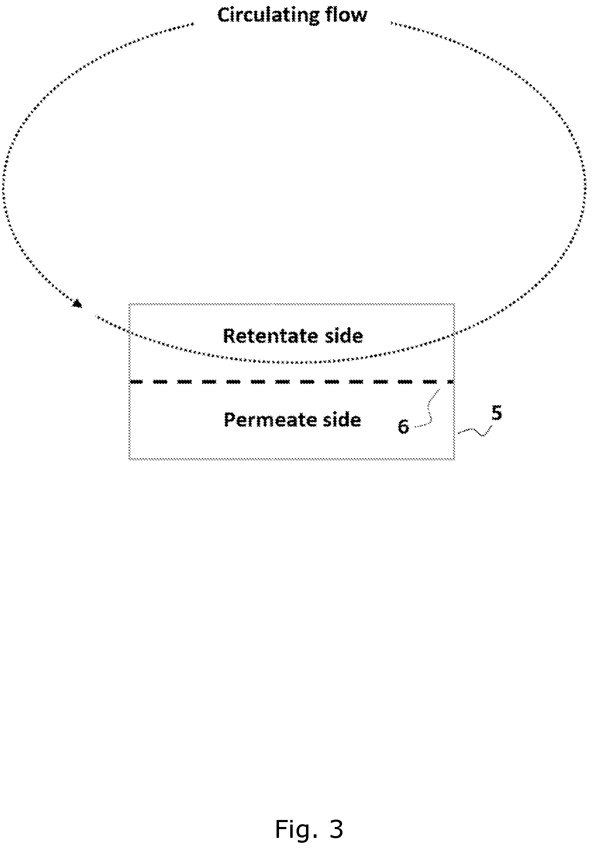

**Figure 3** shows, a schematic representation of the general concept of tangential flow filtration as implemented in the present invention, wherein a mixture to be filtered is flowing over a filter 6 within a tangential flow filtration unit 5 whereby a part of the mixture is retained on the retentate side of the filter as retentate and another part the mixture passes through the filter to the permeate side as permeate. A circulating flow comprising the initial mixture to be filtered and any retentate which has been recirculated back to the tangential flow filtration unit is moving essentially tangential to the filter surface when near to the filter.

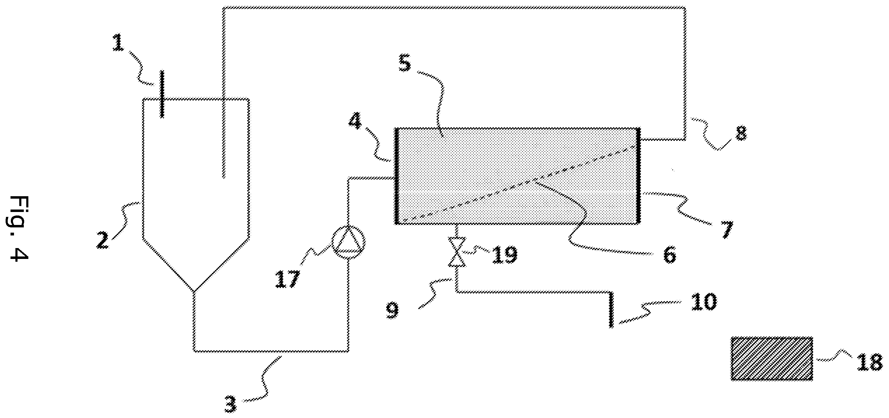

**Figure 4** shows, a schematic representation of an apparatus according to an embodiment of the present invention which is furthermore equipped with a peristaltic pump 17 in a first fluid connection 3 and an opening-closing valve 19 that is controlled by an electronic control unit 18.

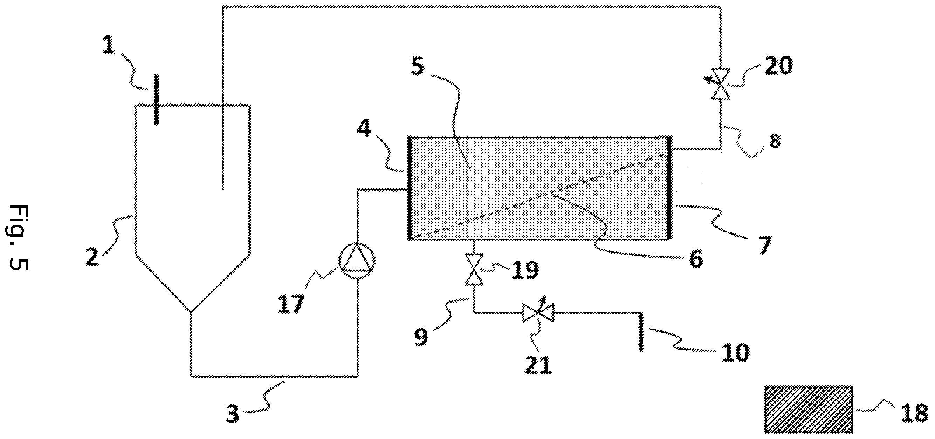

**Figure 5** shows, a schematic representation of an apparatus according to an embodiment of the present invention which, in addition, comprises a first variable flow control valve 20 in a second fluid connection 8 and a second variable flow control valve 21 in a third fluid connection 9. An electronic control unit 18 may control the two variable flow control valves and an opening-closing valve 19.

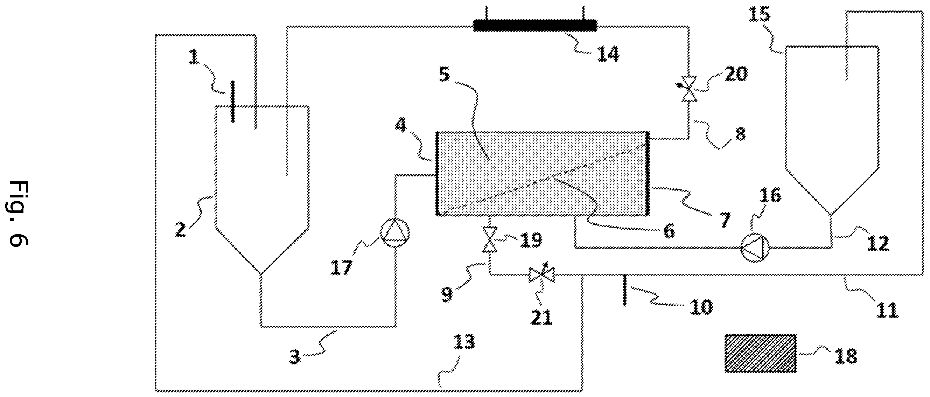

**Figure 6** shows, a schematic representation of an apparatus according to an embodiment of the present invention which is particularly adapted to frequently create events of pulsed pressure increases and/or backward flushes across the filter in the tangential flow filtration unit. This apparatus is further equipped with a fourth fluid connection 11 that leads to a permeate collection tank 15. From this tank a fifth fluid connection comprising a centrifugal pump 16 is leading back to the permeate side of the filter 6. This embodiment, also comprises a sixth fluid connection 13 that connects the third fluid connection 9 with the mixing tank 2, for example for use during a washing step.

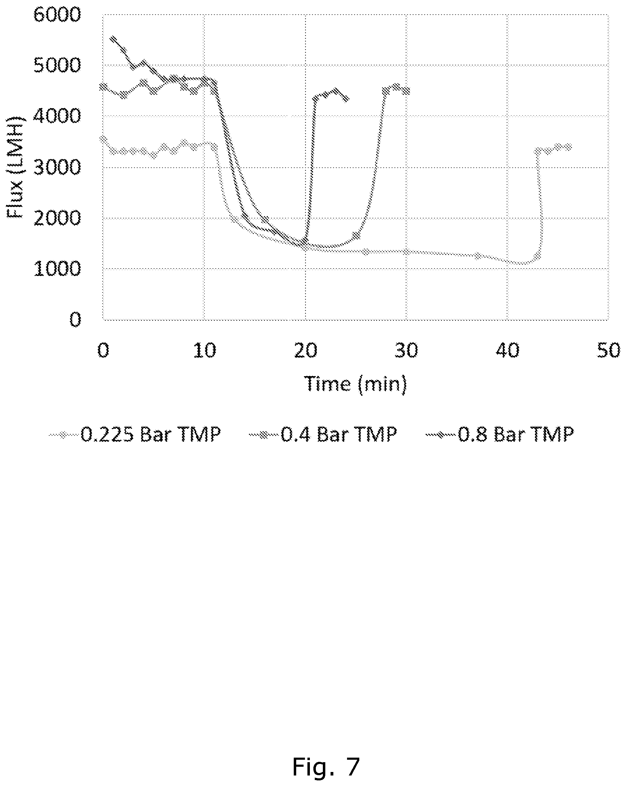

**Figure 7** shows, that the flux of permeate through the filter in the tangential flow filtration unit is high when the apparatus creates frequent events of pulsed pressure increases and/or backward flushes of the permeate. The flux declines rapidly when the backward flush system is turned OFF, however the flux is rapidly restored when the system is turned ON again.

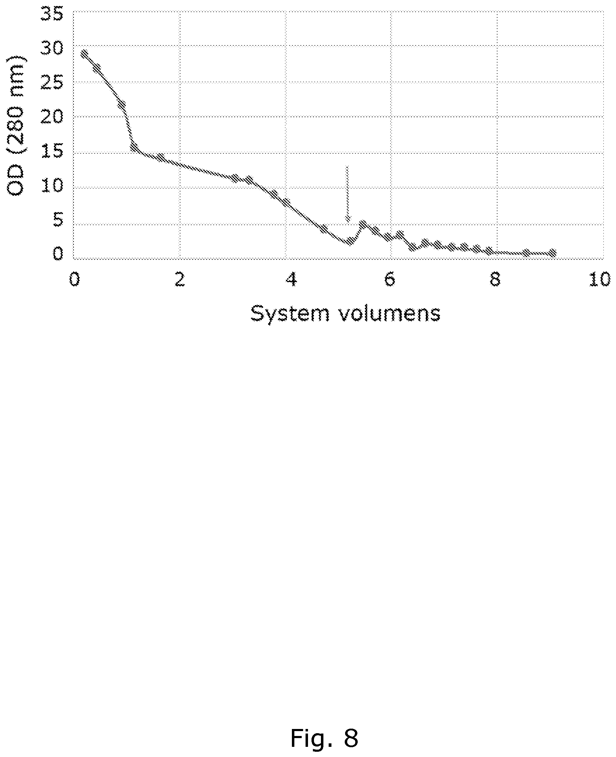

**Figure 8** shows, the optical density (OD) of the permeate measured at 280 nm as a function of system volume (1 system volume = 21 L). The arrow in the figure indicates the point at which elution of the protein is initiated whereby a temporary OD increase is observed as the protein is released from the protein sorption material. About 190 L of water was used for the total process of obtaining a permeate comprising the refined protein and for which the OD reading was decreased by 85% when compared to the initial composition.

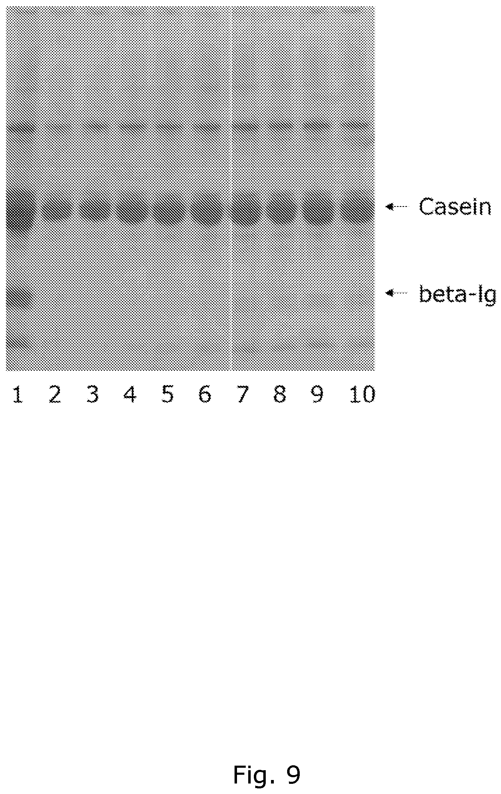

**Figure 9** shows, the SDS-PAGE analysis of the permeate spot samples taken during load of skim milk.

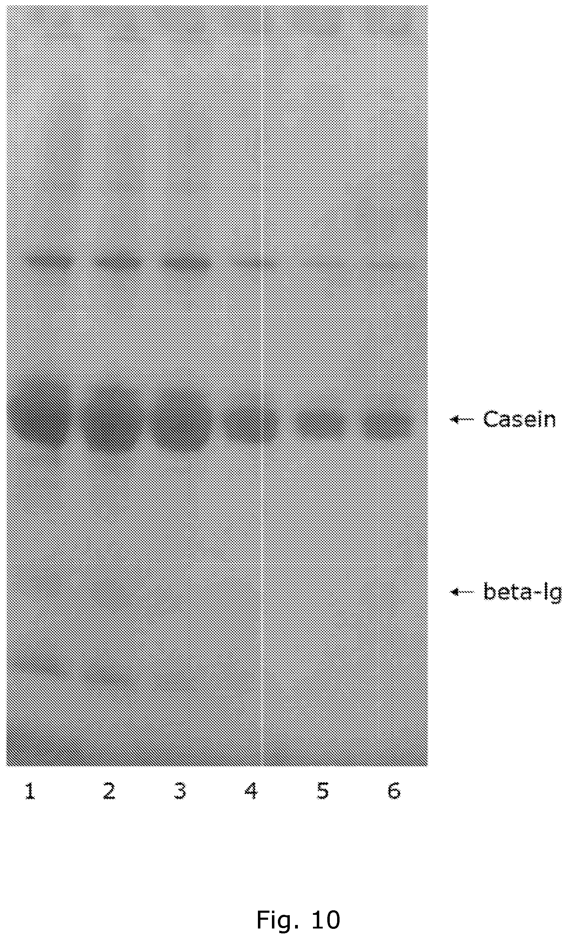

**Figure 10** shows, the SDS-PAGE analysis of the permeate spot samples taken during washout with water until the point at which the elution liquid was added (i.e. the point indicated by an arrow in figure 8).

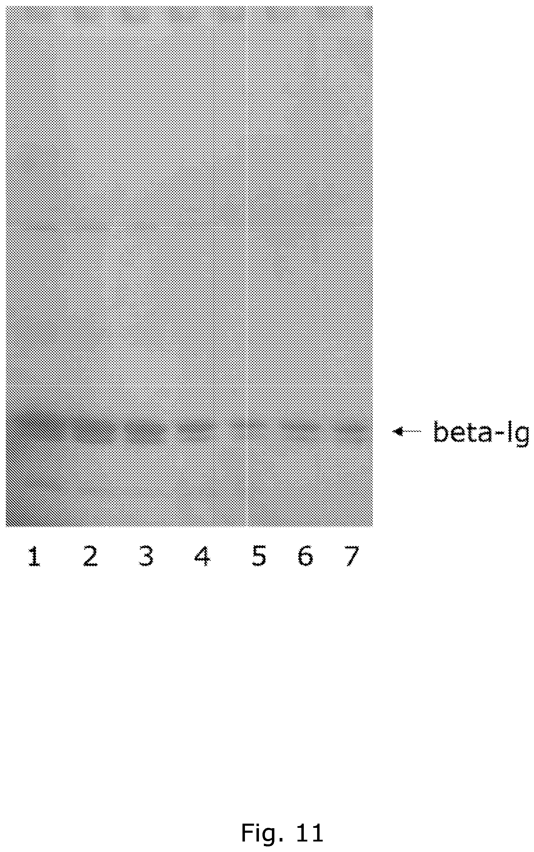

**Figure 11** shows, the SDS-PAGE analysis of the permeate spot samples taken during elution of the bound protein with water at pH 4.5.

The present invention will now be described in more detail in the following.

## Detailed description of the invention

Definitions

Prior to discussing the present invention in further details, the following terms and conventions will first be defined:

*Sorption material*

In the present context, the term "sorption material" refers to a material that is able to attach to a specific compound or group of compounds, such as a proteins or other biomolecules. A sorption material may also be named an adsorbent.

*Adsorbent*

In the present context, the term "adsorbent" refers to a material, which is able to interact with a specific compound or group of compounds which are thereby reversibly attached to the surface of the material. For example, the adsorbent may comprise ligands able to interact with the specific compound, such as but not limited to: ion exchange ligands, hydrophobic ligands, affinity sorption ligands, and mixed mode ligands. An adsorbent may be substantially impermeable whereby only the outer surface is available for interaction with compounds, or it may be highly porous with pores large enough to allow compounds to diffuse into and interact with the inner surface of the adsorbent.
Alternatively, the adsorbent may comprise an immobilized enzyme which is bound by reversible adsorption, physical entrapment or by covalent chemical bonding to the protein sorption material and for enzymatic modification of the compound present in the composition, such as but not limited to controlled hydrolysis of proteins in the composition into peptides by an immobilized protease, or modification of carbohydrates e.g. polysaccharides.

*Elution liquid*

In the present context, the term "elution liquid" refers to a liquid substance or composition, which is capable of releasing a compound attached to a sorption material.

*Circulating flow*

In the present context, the term "circulating flow" refers to the flow of the part of the composition, mixture, liquid, fluid, or suspension that enters the tangential flow filtration unit in the first end and leaves it from the second end as retentate that is circulated back to the first end, such as through the mixing tank.

*Tangential flow filtration unit*

In the present context, the term *"tangential flow filtration unit"* or cross-flow filtration unit, refer to filtration equipment wherein the filter surface is tangential to the flow direction of the composition, which is to be filtered.

*Filter*

In the present context, the term "filter" refers to a filter or a membrane useful for separating components of a composition.

*Flux*

In the present context, the term "flux" or "flux rate" means the volume of liquid passing one square meter of filter area per hour. The unit applied in the art is LMH (i.e. $L/m^2/h$).

*Nominal pore size*

In the present context, the term "nominal pore size" relates to the pore size of a filter at which a minimum percentage of components larger than that pore size should be retained by the filter. For example, a nominal pore size of 60% for a filter having an average pore size of X, is considered to retain at least 60% of particles which are larger than X.

*Retentate*

In the present context, the term "retentate" relates to a composition which has passed over a filter. Thus, a retentate is the part of a filtered composition which was retained by the filter (i.e. retained on the retentate side of the filter, see figure 3).

*Permeate*

In the present context, the term "permeate" relates to a composition which has passed through a filter. Thus, a permeate is the part of a filtered composition which went through the filter (i.e. passed through the filter to the permeate side of the filter, see figure 3).

*Backward flush*

In the present context, the term "backward flush" relates to an event wherein the flow of composition through the filter is reversed.

*Pulsed pressure increase and/or backward flush of the permeate*

In the present context, the term "pulsed pressure increase and/or backward flush of the permeate" relates to an event wherein the pressure on the permeate side is increased. For example, the pressure increase may be large enough that the difference between the pressure on the retentate side and the pressure on the permeate side of the filter is essentially zero, whereby the flow of composition through the filter may be reversed to give a weak backward flush though the filter. In another example, the pressure increase on the permeate side is much larger than the pressure on the retentate side, whereby a strong backward flush is applied.

*Biological composition*

In the present context, the term "biological composition" refers to a complex composition obtained from a biological source, such as plants, microorganisms and animals, and being a liquid, suspension or mixture. For example, the biological composition may be milk, whey, fermentation broth, plant extracts or blood plasma.

*Biomolecules*

In the present context, the term "'biomolecules" refers to molecules that are produced by cells and living organisms.

*Refining*

In the present context, the term "refining" refers to a process wherein a protein or other biomolecule is concentrated, separated, cleaned, purified, and/or isolated from a biological composition.

*Separation*

In the present context, the term "separated" refers to something which has been set apart from something else. More specifically, it may be a protein which has been set apart from other species of a composition, such that a new composition comprising the protein is obtained.

*Transmembrane pressure* (TMP)

In the present context, the term "transmembrane pressure" is defined as:

$$TMP = (feed\ pressure + retentate\ pressure) / 2 - permeate\ pressure.$$

The present invention provides an apparatus for refining a protein comprised in a biological composition.

By reference to figure 1, a first aspect of the present invention relates to an apparatus comprising a first inlet 1 and one or more first fluid connections 3 configure to lead fluid from the inlet to a first end 4 of a tangential flow filtration unit 5 comprising a filter 6 with a nominal pore size in the range of 1 to 150 $\mu$m, and where a second end 7 of the tangential flow filtration unit 5 has a second fluid connection 8 configured to lead retentate back to the first tangential flow filtration unit; the tangential flow filtration unit 5 having a third fluid connection 9 configured to lead permeate to a first outlet 10; and wherein the apparatus comprises an automatic backward flush system being configured to apply a pulsed pressure increase and/or backward flush of the permeate with a frequency in the range of 1-500 per hour.

In figure 2 is an embodiment of the invention shown where the first inlet is connected to a mixing tank 2, and said mixing tank 2 is connected to the first fluid connections 3.

The mixing tank 2 could be of any suitable material and the fluid connections 3 may comprise one or more peristaltic pumps 17. The tangential flow filtration unit 5 may be equipped with any type of filter 6. The third fluid connection 9 configured to lead permeate to outlet is connected to the first outlet 10 that either direct the permeate for collection in a waste tank or in a permeate collection tank.

The first fluid connections 3 and optionally the mixing tank 2 comprises a sorption material for protein or other biomolecule suspended in an aqueous liquid at a concentration of 5 to 70 % by volume. Hence, the sorption material is a part of the apparatus/system of the invention.

The backward flush system is automatic. Thus, in one embodiment the system regulates the frequency and power of the pulsed pressure increases and/or backward flushes as commonly known to the persons skilled in the art.

Figure 4 shows a more detailed schematic representation of an apparatus according to an embodiment of the present invention, wherein the first fluid connection 3 comprises a peristaltic pump 17 and an automatic opening-closing valve 19 in the third fluid connection 9 controlled by an electronic control unit 18. A pulsed pressure increase on the permeate side of the filter may be obtained whenever the opening-closing valve is closed.

Thus, an embodiment of the present invention relates to the apparatus as described herein, wherein the automatic backward flush system comprises:

-   an opening-closing valve 19 in the third fluid connection 9 being controlled by an electronic control unit 18.

A further schematic representation of an apparatus according to an embodiment of the present invention is shown in figure 5 and relates to an apparatus, wherein the apparatus is equipped with a first variable flow control valve 20 in the second fluid connection 8 and a second variable flow control valve 21 in the third fluid connection 9; both valves being controlled by one or more electronic control units 18. The variable flow control valves may be used in conjunction with the opening-closing valve to create a particularly controllable pulsed pressure increase on the permeate side of the filter, such as to obtain a modest backward flush of the permeate.

Thus, an embodiment of the present invention relates to the apparatus as described herein, wherein the automatic backward flush system comprises:

- a first variable flow control valve 20 in the second fluid connection 8 and a second variable flow control valve 21 in the third fluid connection 9, that are being controlled by an electronic control unit 18.

Also, an embodiment of the present invention relates the apparatus as described herein, wherein the automatic backward flush system comprises:

- an opening-closing valve 19 in the third fluid connection 9 and which is being controlled by an electronic control unit 18,
- a first variable flow control valve 20 in the second fluid connection 8 and a second variable flow control valve 21 in the third fluid connection 9, and that are being controlled by an electronic control unit 18.

Figure 6 shows a particularly detailed schematic representation of an apparatus according to an embodiment of the present invention. The permeate and/or the refined product composition can be collected from the outlet, such as in a permeate collection tank. Thus, an embodiment of the present invention relates to the apparatus as described herein, wherein the first outlet 10 is coupled to a permeate collection tank 15 through a fourth fluid connection 11. The same or another of the electronic control units 18 may control a centrifugal pump 16 connected to at least one permeate back flush port through one or more fifth fluid connections 12. The centrifugal pump, when activated, reverses the flow of permeate fluid to obtain a particularly effective backward flush of the permeate through the filter 6. Thus, when the centrifugal pump 16 is activated the opening-closing valve 19 at the third fluid connection 9 can be closed while, liquid delivered by the centrifugal pump 16 enters the permeate side of the filter 6 through the fifth fluid connection 12 so that the pressure increases. Also, the variable flow control valves may be used for obtaining more control of the pressure difference between the retentate and the permeate sides of the filter during an event inflicting a pulsed pressure increase and/or backward flush of the permeate. The centrifugal pump 16 is in fluid connection with the permeate tank 15 and the backward flush is performed using permeate that has been collected prior to the backward flush.

The apparatus may also be equipped with monitors to monitor flow rates, temperature and pressure in all fluid connections. The variable flow control valves positioned in the second fluid connection 8 and/or third fluid connection 9 can be used to regulate the flow and thereby also the back pressure drop from the first end 4 to the second end 7 across the length of the tangential flow filtration unit 5 and across the wall of the filter 6 (from retentate to permeate side) in combination with regulation of the flow rate of the peristaltic pumps 17.

Thus, an embodiment of the present invention relates to the apparatus as described herein, wherein the automatic backward flush system comprises:

- a fourth fluid connection 11 configured to lead the permeate from the first outlet 10 to a permeate collection tank 15,
- a fifth fluid connection 12 connecting the permeate collection tank 15 to the permeate side of the filter 6, and
- a centrifugal pump 16 inserted in the fifth fluid connection 12 and which centrifugal pump is also controlled by the electronic control units 18.

A further embodiment of the present invention relates to the apparatus as described herein, wherein the automatic backward flush system comprises:

- an opening-closing valve 19 in the third fluid connection 9 and which is being controlled by an electronic control unit 18,
- a first variable flow control valve 20 in the second fluid connection 8 and a second variable flow control valve 21 in the third fluid connection 9, and that are being controlled by an electronic control unit 18,
- a fourth fluid connection 11 configured to lead the permeate from the first outlet 10 to a permeate collection tank 15,
- a fifth fluid connection 12 connecting the permeate collection tank 15 to the permeate side of the filter 6, and
- a centrifugal pump 16 inserted in the fifth fluid connection 12 and which centrifugal pump is also controlled by the electronic control units 18.

The backward flush system is applying a pulsed pressure increase and/or backward flush of the permeate. The increased pressure and/or backward flush of the permeate helps release any material that may have piled up, such as having created a flux reducing layer/film or having deposited in and on the filter 6. The release of the material allows the circulating flow to remove said material from the filter 6. Removal of the material results in a cleaner filter with an increased throughput (flux) of permeate. Herein, the deposition or piling of material in and on the filter 6 is also referred to by using the term "fouling".

The backward flush system is operating automatically and is preferably controlled by an electronic control unit 18. In one embodiment, the electronic control unit 18 is itself able to determine the required frequency of operation of the backward flush system, such that the flow rate of permeate through the filter 6 is optimized to increase the rate at which product is collected. The frequency of operation may be very high with short durations for each operation performed. Thus, an embodiment of the present invention relates to the apparatus as described herein, wherein the pulsed pressure increase and/or backward flush of the permeate has a pulse duration of between 0.05 to 150 seconds.

An embodiment of the present invention relates to the apparatus, wherein the frequency of applying backward flush during operation is at least 1 per hour, such as at least 2 per hour, such as at least 4 per hour, such as at least 6 per hour, such as at least 8 per hour, such as at least 10 per hour, such as at least 12 per hour, such as at least 15 per hour, such as at least 20 per hour, such as at least 30 per hour, such as at least 40 per hour, such as at least 50 per hour, such as at least 60 per hour. Another embodiment of the present invention relates to the apparatus as described herein, wherein the frequency of backward flush is in the range of 2-400 per hour, such as in the range of 2-300 per hour, such as in the range of 2-200 per hour, such as in the range of 2-150 per hour, such as in the range of 2-100 per hour, such as in the range of 2-80 per hour, such as in the range of 2-60 per hour, such as in the range of 3-60 per hour, such as in the range of 4-60 per hour, such as in the range of 5-60 per hour, such as in the range of 6-60 per hour, such as in the range of 6-50 per hour, such as in the range of 6-40 per hour, such as in the range of 6-30 per hour.

Yet another embodiment of the present invention relates to the apparatus as described herein, wherein the pulse duration is in the range of 0.05 to 150 seconds, such as in the range of 0.1 to 120 seconds, such as in the range of 0.2 to 100 seconds, such as in the range of 0.3 to 80 seconds, such as in the range of 0.3 to 60 seconds, such as in the range of 0.4 to 50 seconds, such as in the range of 0.5 to 40 seconds, such as in the range of 0.5 to 30 seconds, such as in the range of 0.5 to 25 seconds, such as in the range of 0.5 to 20 seconds, such as in the range of 0.5 to 15 seconds, such as in the range of 0.5 to 10 seconds, such as in the range of 0.5 to 8 seconds, such as in the range of 0.5 to 6 seconds, such as in the range of 0.5 to 5 seconds, such as in the range of 1.0 to 5 seconds.

A more specific embodiment of the present invention relates to the apparatus as described herein, wherein the frequency of the backward flush is 2-60 per hour and the pulse duration is in the range of 0.5 to 30 seconds, such as in the range of 0.5 to 15 seconds, such as in the range of 0.5 to 8 seconds, such as in the range of 0.5 to 5 seconds. However, another specific embodiment of the present invention relates to the apparatus as described herein, wherein the frequency of the backward flush is 6-30 per hour and the pulse duration is in the range of 0.5 to 30 seconds, such as in the range of 0.5 to 15 seconds, such as in the range of 0.5 to 8 seconds, such as in the range of 0.5 to 5 seconds.

During operation of the apparatus the pressure difference between the permeate side and the retentate side of the filter ($P_{permeate}$ - $P_{retentate}$) will be negative. However, during a backward flush event the pressure on the permeate side is increased such that the pressure difference is reduced to zero or become positive. When the pressure difference is positive, liquid may pass through the filter from the permeate side and into the retentate side whereby material on the filter due to fouling is released. Yet, when the pressure difference is zero, there will be practically no liquid passing from the retentate to the permeate side. Thus, the inventors of the present invention were surprised to find that the backward flush may still be effective at cleaning the filter, even when some or all backward flush events are performed with a zero pressure difference. Such zero pressure backward flush events are considered to be advantageous since no, or very little, liquid is returned to the retentate side of the filter and particularly gentle and to increase the lifetime and productivity of the filter. The automatic backward flush system is able to self-initiate moderate pulsed pressure increases and/or backward flushes of the permeate in combination with an electronic measuring system determining when to initiate and control the power of each pulse, is therefore considered particularly relevant for use in filtration of complex biological compositions.

Thus, a particular embodiment of the invention relates to the apparatus as described herein, wherein the pressure difference during a backward flush event can be controlled to be zero (such as by closing the opening-closing valve 19). Another particular embodiment of the invention relates to the apparatus as described herein, wherein 5 -100 %, such as 10-100 %, such as 20-100 %, such as 30-100 %, such as 40-100 %, such as 50-100 % of the backward flush events are performed with a zero backward flush pressure difference.

However, for other applications it was found important to apply a positive backward flush pressure difference to achieve an attractive overall filtration flux of the unit. Yet another particular embodiment of the invention relates to the apparatus as described herein, wherein the pressure difference during a backward flush event can be controlled to be in the range of 0.01-20 bar, such as 0.05 - 10 bar, such as 0.1-8.0 bar, such as 0.1-6.0 bar, such as 0.1-5.0 bar, such as 0.1 - 4.0 bar, such as 0.1 - 3.0 bar, such as 0.2 -3.0 bar, such as 0.3 - 3.0 bar, such as 0.4-3.0 bar, such as 0.5-2.5 bar.

## PUMPS

The present invention may provide an apparatus, which is gentle towards the sorption material, whereby the same material may be employed progressively and stay as an integral part of the apparatus. Thus, some of the parts in the apparatus, e.g. any pumps, may be selected such as to inflict minimal wear on the protein sorption material. A notable embodiment of the present invention therefore relates to the apparatus as described herein, wherein the apparatus comprises one or more pumps comprised in one or more of the first fluid connections 3 and/or the second fluid connection 8, said one or more pumps being configured to exert a low shear force to a sorption material when in use. A further embodiment of the present invention relates to the apparatus as described herein, wherein said one or more pumps is a positive displacement pump. Another embodiment of the present invention relates to the apparatus as described herein, wherein the positive displacement pump is a low-shear pump. Further in this regard, the pumps may be single rotor low-shear pumps selected from the group of vane pump, piston pump, progressing cavity pump, screw pump, and peristaltic pump; or selected from the group of multiple rotor low-shear pumps comprising gear pump, lobe pump, circumferential piston pump, and screw pump. An embodiment of the present invention therefore relates to the apparatus as described herein, wherein the positive displacement pump is selected from the group consisting of vane pump, piston pump, progressing cavity pump, screw pump, gear pump, lobe pump, circumferential piston pump, screw pump, and peristaltic pump, preferably peristaltic pump.

In an embodiment of the present invention, the pumps comprised in one or more of the first fluid connections 3 and/or the second fluid connection 8 are positive displacement pumps that exert a low shear force to a protein sorption material, when in use. This is considered an important embodiment of the present invention as the apparatus is circulating the same sorption material around in the fluid connections (and optionally the mixing tank 2) and the retentate side of the filter 6, when in use. The low shear force exerted by the pumps, said fluid connections and other parts, increases the lifetime of the sorption material. Use of the apparatus according to the present invention is therefore considered to be particularly cost-efficient and environmentally friendly.

By low shear force is to be understood that the same sorption material is circulated around in the fluid connections and parts between the mixing tank and the retentate side of the filter, for at least 50 rounds, such as at least 500 rounds, such as at least 1000 rounds, such as at least 5000 rounds, such as at least 10.000 rounds and/or that each particle of sorption material may adsorb a molecule more than 50 times, such as 500 times, such as 1000 times, such as 5000 times, such as 10.000 times without being degraded i.e. at least 90% of the sorption material is within the size range of the sorption material from a start.

## FILTER

### FILTER PORE SIZE

The tendency of fouling of the filter may be reduced by applying filter types with increased porosity allowing a fraction of the material to pass through the filter into the permeate. Increased pore size also allows for increased flux rate and thus, a faster and more cost efficient process. Furthermore, the inventors of the present invention surprisingly found that by reducing the fouling of the filter, the ability of the backward flush system to apply a suitable backward flush for retaining high flux during operation gets strongly enhanced.

Thus, the nominal pore size of the filter should be as large as possible, but still retain the sorption material. An embodiment of the present invention relates to the apparatus described herein, wherein the filter has a nominal pore size in the range of 20 to 300 $\mu$m. A specific embodiment of the present invention relates to the apparatus described herein, wherein the filter has a nominal pore size in the range of 20 to 80 $\mu$m. Yet, a more specific embodiment of the present invention relates to the apparatus described herein, wherein the filter has a nominal pore size in the range of 20 to 80 $\mu$m, such as 20 to 60 $\mu$m, such as 20 to 50 $\mu$m, such as 20 to 30 $\mu$m. The filter may also have a nominal pore size of 20 to 50 $\mu$m, such as 20 to 40 $\mu$m, preferably 20 to 30 $\mu$m. However, for some applications wherein the raw material contains larger insoluble particles the filter has a large nominal pore size. An embodiment of the present invention therefore relates to the apparatus described herein, wherein the filter has a nominal pore size in the range of 20 to 300 $\mu$m, such as 30 to 250 $\mu$m, such as 30 to 200 $\mu$m, such as 40 to 200 $\mu$m, such as 50 to 200 $\mu$m.

### FILTRATION SYSTEM

An embodiment of the present invention relates to the apparatus as described herein, wherein the tangential flow filtration unit 5 is a tubular filtration system or a cassette filtration system. The tangential flow filtration unit should be equipped with a filter providing a high flux of permeate, such as due to filter nominal pore sizes in the micrometre range or due to effective prevention of fouling by the automatic backward flush system. The filter may therefore be classified as a high flux filter (or membrane) and the backward flush system installed to ensure optimum performance of the filter to increase the efficiency of the system.

Thus, an embodiment of the present application relates to the apparatus as described herein, wherein the filter 6 is a high flux filter with a clean water permeate flux of more than 1000 L/m$^2$/h when the TMP is about 0.1-0.9 bar. More specifically, an embodiment of the present application relates to the apparatus as described herein, wherein the filter 6 is a high flux filter with a clean water permeate flux of more than 1000 L/m$^2$/h, such as more than 2000 L/m$^2$/h, such as more than 3000 L/m$^2$/h, such as more than 4000 L/m$^2$/h, such as more than 6000 L/m$^2$/h at a TMP of about 0.1-0.9 bar. The numbers are shown in terms of the amount of clean water (L) passing through one square meter of the filter (m$^2$) per hour. A further embodiment of the present invention relates to the apparatus as described herein, wherein the filter 6 has a clean water permeate flux of more than 1000 L/m$^2$/h when the TMP is 0.5 bar $\pm$ 0.1 bar. An even further embodiment of the present invention relates to the apparatus as described herein, wherein the filter 6 has a clean water permeate flux of more than 2000 L/m$^2$/h when the TMP is 0.5 bar $\pm$ 0.1 bar. Furthermore, an embodiment of the present invention relates to the apparatus as described herein, wherein the filter 6 has a clean water permeate flux of more than 3000 L/m$^2$/h when the TMP is 0.5 bar $\pm$ 0.1 bar. In addition, an embodiment of the present invention relates to the apparatus as described herein, wherein the filter 6 has a clean water permeate flux of more than 4000 L/m$^2$/h when the TMP is 0.5 bar $\pm$ 0.1 bar. However, a precious embodiment of the present invention relates to the apparatus as described herein, wherein the filter 6 has a clean water permeate flux of more than 6000 L/m$^2$/h when the TMP is 0.5 bar $\pm$ 0.1 bar. The uncertainty of the TMP may in some embodiment be as small as 0.05 bar, 0.01 bar, or even 0.001 bar.

The tangential flow filter may be made of any material suitable for a filtration device to be applied in aqueous solutions. Thus, an embodiment of the present invention relates to the apparatus as described herein, wherein the filter 6 is of a filter material selected from the group consisting of porous inorganic material, porous organic polymer, and mixtures thereof.

A filter according to the present invention could have been prepared by a method suitable for creating high flux filters, such as a method for creating high porosity filters. A particular embodiment of the present invention therefore relates to the apparatus as described herein, wherein the filter material has been melt blown, spun, woven, sintered or cast to create the target porosity of the filter 6.

*FILTER AND FILTER DEVICE*

The filter is of a porous inorganic material and/or porous organic polymer material. A specific embodiment of the present invention therefore relates to the apparatus as described herein, wherein the filter 6 is of a filter material selected from the group consisting of sintered metal, sintered metal powder, sintered metal strings and layers hereof, sintered metal oxides, sintered ceramics, porous glass, polypropylene, polyethylene, cellulose, cellulose acetate, viscose, polysulfone (PSU), polyethersulfone (PES), polyarylethersulfone (PAES), polyvinylidinedifluoride (PVDF), polytetraflouorethylene (PTFE), polyamide, nylon, polyester, surface modified PES, surface modified PTFE, and mixtures thereof. An embodiment of the present invention relates to the apparatus as described herein, wherein the filter material is porous sintered stainless steel particles or porous sintered stainless steel mesh.

A further embodiment of the present invention relates to the apparatus as described herein, wherein the filter is of a porous organic polymer material selected from the group consisting of polypropylene, polyethylene, cellulose, cellulose acetate, viscose, polysulfone (PSU), polyethersulfone (PES), polyarylethersulfone (PAES), polyvinylidinedifluoride (PVDF), polytetraflouorethylene (PTFE), polyamide, nylon, polyester, surface modified PES, surface modified PTFE, and mixtures thereof.

A further particular embodiment of the present invention relates to the apparatus as described herein, wherein the filter is a polypropylene filter. Another particular embodiment of the present invention relates to the apparatus as described herein, wherein the filter is a polyethylene filter. Yet another particular embodiment of the present invention relates to the apparatus as described herein, wherein the filter is a polyamide filter, such as a nylon filter. Just another particular embodiment of the present invention relates to the apparatus as described herein, wherein the filter is a melt blown polypropylene filter.

The filters according to the present invention may have a variety of shapes and forms. Particularly relevant are filters designed for cross-flow filtration wherein the retentate liquid is passing the filtering surface in a direction perpendicular to the direction of the filtrate/permeate flow. Cross-flow filtration devises are often also named tangential flow filtration devices, as opposed to dead-end filtration devices wherein the liquid is passing directly through the filtering surface. Cross-flow filtration devices may be in the form of hollow fibers, tubular or cylindrical, and they may be in the form of flat sheets, discs or plates. Flat sheet cross-flow membranes may be spiral wound or they may be stacked with spacers in between each membrane layer. The cross-flow filters may further be designed for maximum flux and stabilization of flux by vibration, shaking, rotation or spinning. In an embodiment of the present invention the filter unit is designed for applying backward flushes during operation of the filtration process. Also, the filter and the filtration device may be designed for hygienic operations enabling efficient cleaning of the system before and after operation.

An embodiment of the present invention relates to the apparatus as described herein, wherein the filter 6 is a hollow fibre filter, tubular or cylindrical, or flat sheet filter.

In a particular embodiment of the present invention relating to the apparatus as described herein, the filter 6 is a hollow fibre filter or a tubular or cylindrical filter. Another particular embodiment of the present invention relates to the apparatus as described herein, wherein the filter 6 is a spiral wound flat sheet filter or a stacked flat sheet filter.

For reasons of their surprisingly lower tendency to get blocked by the adsorbent during operation, and their stability towards back flushing and back pulsing it is particularly preferred that the filter is a hollow fibre or a tubular or cylindrical filter.

When the filter is a tubular hollow fibre filter or cylindrical hollow fibre filter, the inner diameter of the filter should be in the range of 2-80 mm. An embodiment of the present invention therefore relates to the apparatus as described herein, wherein the inner diameter of the hollow fibre filter is in the range of 2-80 mm, such as 3-50 mm, 4-50 mm, such as 4-40 mm, such as 5-40 mm, such as 5-35, such as 6-35 mm, such as 6-30 mm, such as 6-25 mm, such as 6-20 mm, such as 6-15 mm, such as 6-12 mm. However, a particular embodiment of the present invention relates to apparatus as described herein, wherein the filter 6 is a tubular hollow fibre filter having an inner diameter in the range of 6-12 mm.

A further advanced embodiment of the present invention relates to the apparatus as described herein, wherein hollow fibre filter is tubular and coiled with a coil outer diameter in the range of 3-40 cm, such as 4-35 cm, such as 5-30 cm, such as 7-25 cm, such as 10-20 cm.

Yet another embodiment of the present invention relates to the apparatus as described herein, wherein the filter 6 is a tubular hollow fibre filter with an inner diameter in the range of 6-12 mm and a length of the liquid path from inlet to retentate outlet in the range of 25-100 cm, such as in the range of 30-90 cm, such as in the range of 35-80 cm, such as in the range of 35-70 cm. An additional embodiment of the present invention relates to the apparatus as described herein, wherein the filter 6 is a tubular hollow fibre filter with an inner diameter in the range of 3-6 mm and a length of the liquid path from inlet to retentate outlet in the range of 5-30 cm, such as in the range of 5-25 cm, such as in the range of 5-20 cm.

When the filter is a spiral wound flat sheet filter or stacked flat sheet filter having spacers between the sheets, the spacer thickness may be in the range of 2-50 mm. However, the spacer thickness is critical for reducing the risk of blocking the tangential flow fluid path in flat sheet membrane systems when high adsorbent concentrations are employed. An embodiment of the present invention therefore relates to the apparatus as described herein, wherein the flat sheet filter has spacers between the sheets and the spacers have a thickness of 2-50 mm, such as 3-50 mm, such as 4-50 mm, such as 5-50 mm, such as 6-50 mm, such as 7-50 mm, such as 8-50 mm, such as 10-50 mm.

In a particular embodiment of the present invention the filter is a flat sheet cassette being vibrated or shaked during operation.

Furthermore, the filters may for some applications be depth filters, wherein the filter sheets and hollow fibres are thick. Thus, a particular embodiment of the present invention relates to the apparatus as described herein, wherein the filter 6 is a depth filter with a wall thickness of 1-50 mm. In a more specific embodiment of the present invention, the filter 6 is a depth filter with a wall thickness of 1-40 mm, such as 1-30 mm, such as 1-20 mm, such as 1.5-20 mm, such as 2-20 mm, such as 3-20 mm, such as 3-15 mm, such as 3-10 mm, such as 4-20 mm, such as 5-20 mm, such as 6-20 mm, such as 8-20 mm.

The length of the liquid path from inlet to retentate outlet of the tangential flow filtration unit is considered important when the concentrations of protein sorption material in the circulating flow is high. Thus, an embodiment of the present invention relates to the apparatus as described herein, wherein the length of the liquid path from the first end 4 to the second end 7 of the tangential flow filtration unit 5 is in the range of 10-150 cm, such as in the range of 15-120 cm, such as in the range of 20-110 cm, such as in the range of 25-100 cm, such as in the range of 30-90 cm, such as in the range of 35-80 cm, such as in the range of 35-70 cm.

## SORPTION MATERIAL

An aspect of the present invention relates to the apparatus as described herein, wherein the apparatus comprises a sorption material for protein or other biomolecule. The sorption material is preferably protein sorption material. An embodiment of the present invention relates to the apparatus as described herein, wherein the sorption material has a particle size distribution such that 90 % by volume of the particles has a diameter in the range of 10-1000 $\mu$m. A further embodiment of the present invention relates to the apparatus as described herein, wherein the sorption material has a particle size distribution such that 90 % by volume of the particles has a diameter in the range of 10-1000 $\mu$m, such as in the range of 10-700 $\mu$m, such as in the range of 10-500 $\mu$m, such as in the range of 10-400 $\mu$m, such as in the range of 10-300 $\mu$m such as in the range of 10-250 $\mu$m, such as in the range of 10-200 $\mu$m, such as in the range of 10-150 $\mu$m, such as in the range of 10-100 $\mu$m, such as in the range of 10-80 $\mu$m, such as in the range of 10-70 $\mu$m, such as in the range of 10-60 $\mu$m, such as in the range of 10-50 $\mu$m, such as in the range of 10-40 $\mu$m. However, for some applications it may be an advantage to employ a sorption material of larger particle size. Thus, an embodiment of the present invention suitable for such application relates to the apparatus as described herein, wherein the sorption material has a particle size distribution such that 90 % by volume of the particles has a diameter in the range of 30-1000 $\mu$m such as in the range of 30-700 $\mu$m, such as in the range of 30-500 $\mu$m, such as in the range of 30-400 $\mu$m, such as in the range of 30-300 $\mu$m such as in the range of 30-250 $\mu$m, such as in the range of 30-200 $\mu$m, such as in the range of 30-150 $\mu$m, such as in the range of 30-100 $\mu$m, such as in the range of 30-90 $\mu$m, such as in the range of 30-70 $\mu$m; However, the particle size distribution may also be in the range of 40-250 $\mu$m, such as in the range of 50-250 $\mu$m, such as in the range of 50-200 $\mu$m, such as in the range of 50-150 $\mu$m.

In yet another embodiment of the present invention, the sorption material is highly porous in order to allow migration and binding of molecules to the material surfaces presented inside the volume of the particles. Thus, in an embodiment of the invention the sorption material is porous and at least 90 %, such as 95 %, such as 98 % and preferably at least 99 % of which have a lower pore diameter limit of 10 nm and an upper pore diameter limit of 200 nm. Thus, a pore diameter between 10 and 200 nm is understood to mean that at least 90 %, such as 95 %, such as 98 %, and preferably at least 99 % of all pores have a pore diameter between 10 and 200 nm. The pores have a lower pore diameter limit of 20 nm and an upper pore diameter limit of 150 nm, preferably 100 nm, more preferably the pores have a lower pore diameter limit of 30 nm and an upper pore diameter limit of 100 nm, preferably 75 nm. The pore diameter is conveniently determined by porosimetry using mercury intrusion.

A porous sorption material in this respect is a material in which the porosity, defined as the total pore volume of all pores with a diameter smaller than 150 nm is between 0.5 ml/g and 1.5 ml/g, such as in the range 0.7-1.1 ml/g. Porosity, with respect to this invention, is also determined by mercury intrusion.

In order for the proteins or other biomolecules of the biological composition to be attached to the sorption material, said material should have surface characteristics that enables efficient interaction with molecules of protein or other biomolecules. The base material of the sorption material may have suitable binding sites (ligands) already present when the sorption material particles are formed, or the ligands may be introduced and covalently coupled to the material of particles after the initial formation.

The type of sorption material to be utilized depends on the nature of the protein or other biomolecules to be separated from the biological composition. The sorption material should preferably get attached to the target protein or other biomolecules to form a conjugate-like structure (such as by adsorption). The conjugate carries the protein or other biomolecules around in the circulating flow, such that the conjugate of sorption material and biomolecule (e.g. protein) passes over the retentate side of the filter several times, each time allowing impurities in the circulation flow to pass through the filter to the permeate side and into a waste tank or permeate collection tank. The conjugate of sorption material and biomolecule may be further cleaned and isolated by use of an installed washing system applying one or more washing liquids to the circulating flow. Whenever, a desired concentration and purity have been reached an elution liquid is added to the circulating flow, whereby the target protein or other biomolecule is released from the sorption material. The liquid composition comprising the target protein or other biomolecule then passes through the filter to the permeate side and may then be collected from an outlet or collected in the permeate collection tank.

The apparatus may be used for upgrading a large scope of biological compositions and/or concentration and purification of proteins typically comprised therein. An embodiment of the present invention relates to the apparatus as described herein, wherein the sorption material is able to adsorb one or more proteins or other biomolecule of a biological composition selected from the group consisting of mammalian milk or whey, such as bovine milk, sheep milk, goat milk or camel milk, whey, bovine blood plasma, porcine blood plasma, human blood plasma, egg white, egg yolk, fermentation broth, fermentation broth of genetically modified microorganisms, fermentation broth of mammalian cell cultures, animal tissue extract, plant tissue extract, algae extract, potato tuber extract, and legume extract.

A further advanced embodiment of the present invention relates to the apparatus as described herein, wherein the sorption material is able to adsorb one or more proteins or other biomolecules of a biological composition, the biological composition being:

- bovine milk, sheep milk, goat milk, or camel milk or whey or a derivative thereof comprising at least one of said proteins selected from the group consisting of lactoferrin, immunoglobulin G, alpha-lactalbumin, and beta-lactalbumin, or
- bovine, porcine, or human blood plasma or a derivative hereof comprising at least one of said proteins selected from the group consisting of albumin, transferrin, immunoglobulin G, immunoglobulin A, alpha-1-antitrypsin, and Factor VIII, or
- egg white or egg yolk or a derivative or mixture hereof comprising at least one of said proteins selected from the group consisting of ovalbumin, lysozyme, immunoglobulin Y, ovomucoid, ovotransferrin, and avidin, or
- plant tissue, potato or legume extract comprising at least one of said proteins or other biomolecules selected from the group consisting of patatin, protease inhibitor, legumin, vicilin, peptides, flavonoids, polyphenols, carbohydrates, polysaccharides, vitamins, phytosterols and glycoalkaloids, or
- fermentation broth of genetically modified microorganisms or a derivative thereof comprising at least one of said proteins selected from the group consisting of cheese rennet, such as bovine chymosin, camel chymosin; enzymes such as amylases, lipases, pollunases, pectinases, xylases, laccases, transglutaminases, phytases, proteases, oxidoreductases, cellulase, chitinase; functional proteins for gelling, emulsification or foaming in food/feed formulations such as rubisco, patatin, legumin, vicilin, protease inhibitor, beta-lactoglobulin, alfa-lactalbumin, ovalbumin, albumin; proteins or peptides having antimicrobial activity such as lactoferrin, transferrin, ovotransferrin, lactoperoxidase, lysozyme, avidin, nicin; sweet tasting proteins or peptides such as thaumatin, brazzein, curculin, mabinlin, monellin, pentadin, antifreeze proteins including but not limited to antifreeze proteins from plants, fish or other marine animals, and any modified and/or engineered forms of such proteins.

In an embodiment of the present invention relating to the apparatus as described herein, the sorption material is an inorganic sorption material or a polymer sorption material or a composite thereof.

The sorption material may comprise, as the backbone, any natural or synthetic and organic or inorganic material known *per se* to be applicable in adsorption based separation of proteins or other biomolecules, e.g. natural or synthetic polysaccharides such as agar-agar and agaroses; celluloses, cellulose ethers such as hydroxypropyl cellulose, carboxymethyl celluose; starches; gums such as guar gum, and gum arabic, gum ghatti, gum tragacanth, locust bean gum, xanthan gum; pectins; mucins; dextrans; chitins; chitosans; alginates; carrageenans; heparins; gelatins; synthetic polymers such as polyamides such as polyacrylamides and polymethacrylamides; polyimides; polyesters; polyethers; polymeric vinyl compounds such as polyvinylalcohols and polystyrenes; polyalkenes; inorganic materials such as silicious materials such as silicon dioxide including amorphous silica and quartz; silicas; metal silicates, controlled pore glasses and ceramics; metal oxides and sulfides, or combinations of these natural or synthetic and organic or inorganic materials.

An embodiment of the present invention relates to the apparatus as described herein, wherein the sorption material is a polymer sorption material comprising one or more polymer selected from the group consisting of natural polysaccharide, agar-agar, agarose, dextran, cellulose, cellulose ethers, hydroxypropyl cellulose, carboxymethyl celluose, starches, gum, guar gum, gum arabic, gum ghatti, gum tragacanth, locust bean gum, xanthan gum, pectin, mucin, chitin, chitosan, alginate, carrageenan, heparin, gelatin, synthetic polymer, polyamide, polyacrylamide, polymethacrylamide, polyimide, polyacrylate, polymeric vinyl, polystyrene, polyalkene, polyvinyl alcohol, polyester, polyether, silicious material, silicon dioxide, amorphous silica, quartz, silica, metal silicate, controlled pore glass, ceramic, metal oxide, sulphide, and composites thereof.

Especially interesting materials as matrix backbones for the sorption material are e.g. agar or agarose beads such as Sepharose and Superose beads from Cytiva, USA, Agarose Beads from ABT, Spain and Biogel A from Biorad, USA; dextran based beads such as Sephadex, Cytiva, USA; cellulose based beads and membranes such as Perloza cellulose Czech Republic, composite beads such as Sephacryl and Superdex, Cytiva, USA; beads of synthetic organic polymers such as Fractogel from Toso-Haas, USA.

A particular embodiment of the present invention therefore relates to the apparatus as described herein, wherein the sorption material is a polymer sorption material comprising one or more polymer selected from the group consisting of natural polysaccharides, agar-agar, agarose, dextran, cellulose, synthetic polymer, and composites thereof.

A specific embodiment of the present invention relates to the apparatus as described herein, wherein the synthetic polymer is selected from the group consisting of polyacrylamide, polyacrylate, poly(allylamine), poly(allylalcohol), poly(vinylamine), poly(vinylalcohol), and composites thereof.

Typically, the sorption phase may e.g., be in the form of irregular particles, fibers or spherical beads. The solid phase material may further be fully or partly permeable or completely impermeable to proteins and other biomolecules. An embodiment of the present invention relates to the apparatus as described herein, wherein the protein sorption material is in the form of beads, pellets, particles, fibres, or grains.

Also, the sorption material should preferably be resistant to shear forces so that the sorption material is not degraded into entities with diameters that allow the material to pass through the filter. An embodiment of the present invention therefore relates to the apparatus as described herein, wherein the sorption material is resistant to shear forces. Furthermore, the sorption material of the present invention is selected such that the material is resistant to shear forces while also having a porous structure. An embodiment of the present invention therefore relates to the apparatus as described herein, wherein the sorption material is a porous adsorption material.

The sorption material may be designed to perform separation and isolation of proteins and other biomolecules in a range of different reversible adsorption procedures, such as ion exchange chromatography, and biospecific affinity chromatography, such as immunosorption and protein A chromatography, and group specific affinity chromatography, such as hydrophobic, thiophilic, mixed mode, dye, lectin, and metal chelate chromatography.

The sorption material may have been obtained by reacting the sorption material used as the backbone for the sorption material with any compound that after reaction provides a sorption material having ligands that interact, such as may bind to the target protein or other biomolecule to be separated from the biological composition.

Such interactions are, for example, the presence of negatively or positively charged groups covalently linked to, or being a natural integral part of the sorption material. Examples of groups that may form negatively charged groups, depending on pH in the medium, are carboxylic acids, sulfonic acids, and phosphonic acids. Examples of groups that may form positively charged groups, depending on pH in the medium, are primary amines, secondary amines, tertiary amines such as the diethylaminoethyl- group (DEAE) and quaternary amines, such as the diethyl-(2-hydroxypropyl)aminoethyl-group (QAE). These charged sorption materials may be broadly used for separation and isolation of proteins and other biomolecules by the ion exchange chromatography method.

An embodiment of the present invention therefore relates to the apparatus as described herein, wherein the sorption material comprises one or more type of ligand selected from the group consisting of ion exchange ligand, affinity sorption ligand, and mixed mode ligand. More specifically, an embodiment of the present invention relates to the apparatus as described herein, wherein the protein sorption material is an affinity sorption material. In this regard, an embodiment of the present invention relates to the apparatus as described herein, wherein the sorption material is an affinity sorption material selected from the group consisting of metal chelate adsorption material, hydrophobic interaction adsorption material, thiophilic interaction adsorption material, mixed mode interaction material, protein A adsorption material, and immobilized antibody adsorption material.

Hydrophobic interactions between the sorption material and proteins or other biomolecules to be isolated may, for example, be achieved if the material surface has hydrophobic groups such as phenyl- groups, hydrocarbons such as octyl-, hexyl or butyl- groups. Thiophilic interactions may, for example, be achieved if the material surface is activated with divinyl sulfone and coupled with mercaptoethanol or 4-hydroxy-pyridine, 3-hydroxy-pyridine, 2-hydroxy-pyridine. Mixed mode interactions may, for example, be achieved if the material surface has groups that can be either non-charged and hydrophobic or positively or negatively charged, dependent on pH in the medium, such as benzylamines; alkylamines, such as hexyl- and octyl-amines; benzoic acids, such as mercaptobenzoic acids and amino-benzoic acids; phenyl-alkyl carboxylic acids, aminophenyl-alkyl carboxylic acids.

The ligands may be attached to the sorption material by any type of bond, either by a direct chemical reaction between the ligand and the sorption material used as the backbone for the sorption material or by activation of the ligand with a suitable reagent making it possible to link the backbone material and the ligand. Examples of such suitable activating reagents are epichlorohydrin, epibromohydrin, allyl bromide, allyl glycidylether; bis-epoxides such as butanediol diglycidylether; halogen-substituted aliphatic compounds such as di-chloro-propanol, divinyl sulfone; carbonyldiimidazole; aldehydes such as glutaric dialdehyde; quinones; cyanogen bromide; periodates such as sodium-meta-periodate; carbodiimides; chloro-triazines such as cyanuric chloride; sulfonyl chlorides such as tosyl chlorides and tresyl chlorides; N-hydroxy succinimides; 2-fluoro-1-methylpyridinium toluene-4-sulfonates; oxazolones; maleimides; pyridyl disulfides; and hydrazides. Among these, the activating reagents leaving a spacer group different from a single bond, e.g. epichlorohydrin, epibromohydrin, allyl-glycidylether; bis-epoxides; halogen-substituted aliphatic compounds; divinyl sulfone; aldehydes; quinones; cyanogen bromide; chloro-triazines; oxazolones; maleimides; pyridyl disulfides; and hydrazides. Especially interesting activating reagents are believed to be epoxy-compounds such as epichlorohydrin, allyl-glycidylether and butanediol diglycidylether. A particular embodiment of the present invention relates to the apparatus as described herein, wherein the protein sorption material has ligands obtained by reaction with a reagent selected from the group consisting of epichlorohydrin, allyl-glycidylether, benzylamine, glycidyl-trimethyl-ammonium chloride, and butanediol diglycidylether.

The ligand concentration on the sorption material may have a strong influence on the binding capacity of the material. If the ligand concentration is too low then only small amounts of protein or other biomolecules may be bound per liter of the sorption material. However, if the ligand concentration is too high it may also lead to more or less irreversible binding of the protein or other biomolecules which will lead to unwanted product losses and poor economic performance of the process. In the context of the present invention the binding capacity is defined as the amount of biomolecule (expressed as gram dry biomolecule) that can be bound to one liter of a sorption material which is fully hydrated but drained for interstitial water by suction drying on a sintered, porous glass funnel. In an embodiment of the present invention relating to the apparatus as described herein, the protein sorption material has a binding capacity in the range of 1-250 g/L sorption material, such as 5-200 g/L, such as 10-200 g/L, such as 20-200 g/L, such as 25-200 g/L, such as 30-200 g/L, and such as 40-200 g/L.

The optimal ligand concentration may depend on the specific application, the concentration of protein or other biomolecule in the biological composition and other process specific parameters. In the context of the present invention the ligand concentration is defined as the amount of ligand expressed in millimoles (i.e. $10^{-3}$ mole) that is attached to one liter of a sorption material which is fully hydrated but drained for interstitial water by suction drying on a sintered, porous glass funnel.

An embodiment of the present invention relates to the apparatus as described herein, wherein the ligand concentration on the sorption material is in the range of 1-3000 millimoles/L of sorption material, such as 10-2500 millimoles/L, such as 20-2500 millimoles/L, such as 30-2500 millimoles/L, such as 40-2500 millimoles/L, such as 50-2500 millimoles/L, such as 60-2500 millimoles/L, such as 80-2500 millimoles/L, such as 100-2500 millimoles/L, such as 125-2500 millimoles/L, such as 150-2500 millimoles/L, and such as 200-250 millimoles/L.

For certain applications, the biomolecules, e.g. protein binds very strongly to the sorption material and then the ligand concentration should be in the range of 1-300 millimoles/L. Thus, a particular embodiment of the present invention relates to the apparatus as described herein, wherein the ligand concentration on the sorption material is in the range of 1-300 millimoles/L, such as 5-250 millimoles/L, such as 10-225 millimoles/L, such as 20-200 millimoles/L, such as 30-180 millimoles/L, such as 30-150 millimoles/L, such as 30-120 millimoles/L, such as 30-100 millimoles/L, such as 30-80 millimoles/L, such as 30-60 millimoles/L. However, particularly the ligand concentration on the protein sorption material may be in the range of and such as 40-150 millimoles/L, 40-120 millimoles/L, 40-100 millimoles/L, 40-80 millimoles/L.

During use of the apparatus, the sorption material will often be exposed to a wide range of pH values, harsh chemicals and even high temperatures. It is therefore important that the sorption material is stable under such conditions and that it does not erode into smaller particles, gets solubilized, or obtains an altered and undesired porous structure. For example, the sorption material should preferably be resistant to shear forces. Such a resistant and stable sorption material may be obtained through chemical cross-linking of the sorption material backbone by methods generally used for stabilization of chromatographic adsorbents. Thus, an embodiment of the present invention relates to the apparatus as described herein, wherein the sorption material is cross-linked.

Examples of suitable cross-linking agents are epichlorohydrin, boric acid, epibromohydrin, allyl bromide, allyl glycidylether; bis-epoxides such as butanediol diglycidylether; halogen-substituted aliphatic compounds such as di-chloro-propanol, divinyl sulfone, diacrylates, diacrylamides such as piperazine-diacrylamide, bis-acrylamides such as N,N-methylene-bisacrylamide, and mixtures and derivatives thereof. A particular embodiment of the present invention relates to the apparatus as described herein, wherein the sorption material has been cross-linked with a reagent selected from the group consisting of epichlorohydrin, butanediol diglycidylether, diacrylates, bis-acrylamide, allyl bromide, allyl glycidylether, boric acid, and mixtures and derivatives thereof.

The mixing tank, the retentate side of the filter unit and the fluid connections enabling recirculation of the sorption material constitutes some of the elements of the apparatus wherein the sorption material is distributed during use of the apparatus. The total volume of the fluid wherein the sorption material is distributed is herein defined as the "system volume". The system volume is not a fixed volume since the mixing tank may be filled to different degrees and the volume may also be adjusted during operation. The sorption material will be suspended in the fluid at a concentration which is measured as the relative volume of packed sorption material achieved when a sample of the suspended material is centrifuged at 3000 G for 5 min expressed in percent of the total suspension sample volume. Thus, if a 100 ml sample of the suspended sorption material is withdrawn from a well-mixed suspension in the mixing tank and centrifuged and the packed bed of sorption material is determined to have a volume of 33 ml, then the concentration of sorption material is 33/100 x 100 % = 33 %.

The concentration of sorption material in the system volume is critical for the total water consumption per kg biomolecule, e.g. protein, produced of any biomolecule separation process performed with the apparatus. The total water consumption per kg biomolecule produced is defined as the volume of aqueous fluids added to the apparatus during the washing and elution steps of the process according to the invention per kg biomolecule (on a dry matter basis) eluted during one cycle of the process. Accordingly, the water consumption per kg biomolecule produced will depend on the concentration of the sorption material in the system volume. At low concentrations the water consumption will be high and at high concentrations of the sorption material the water consumption per kg biomolecule produced will be relatively lower.

Thus, an embodiment of the present invention relates to the apparatus as described herein, wherein the sorption material is suspended in an aqueous liquid. A further embodiment of the present invention relates to the apparatus as described herein, wherein the circulating flow that enters the tangential flow filtration unit 5 in the first end 4 and leaves it from the second end 7 as retentate being circulated back to the first end 4, comprises the sorption material in a concentration in the range of 1-70 %, such as in the range of 3-70 %, such as in the range of 5-70 %, such as in the range of 7-65 %, such as in the range of 10-65 %, such as in the range of 15-65 %, such as in the range of 20-65 %, such as in the range of 25-65 %, such as in the range of 30-65 %, such as in the range of 35-65 %, and such as in the range of 40-65 %.

For some applications it may further be advantageous to use different concentrations of the sorption material during the different steps of a adsorption cycle. Thus, an embodiment of the present invention relates to the apparatus as described herein, wherein the concentration of the sorption material during loading of the biological to the mixing tank is in the range of 1-30 %, such as in the range of 3-30 %, such as in the range of 5-30 %, such as in the range of 5-25 %, such as in the range of 7-25 %, and such as in the range of 10-25 %. In another embodiment of the present invention relating to the apparatus as described herein, the concentration of the sorption material during wash and/or elution is in the range of 20-70 %, such as in the range of 25-70 %, such as in the range of 25-65 %, such as in the range of 30-65 %, and such as in the range of 40-65 %.

The binding capacity of the sorption material is likewise of critical importance for the total liquid consumption per kg biomolecule produced of any biomolecule separation process performed with the apparatus. If the binding capacity is low, the volume of aqueous fluids added to the apparatus during the washing and elution steps of the process according to the invention per kg biomolecule (on a dry matter basis) during one cycle of the process will be high, and thereby the waste handling and cost of the process may become too high.

Thus, an embodiment of the present invention relates to the apparatus as described herein, wherein the sorption material has a binding capacity for the protein or other biomolecule to be refined of at least 5 g/L adsorbent, such as at least 10 g/L adsorbent, such as at least 15 g/L, such as at least 20 g/L, such as at least 25 g/L, such as at least 30 g/L. such as at least 35 g/L, such as at least 40 g/L adsorbent on a dry matter basis.

In a particular embodiment of the present invention the sorption material is initially present in the first fluid connections 3 and/or mixing tank as a suspension in water or other suitable aqueous solution before the biological composition is added. In this case the biological composition comprising the target protein or other biomolecule may then be added (feeded) gradually by a feeding pump while the sorption material is mixed and recirculated through the tangential flow filtration unit and the first permeate is taken out (bleeded) with a flow rate which is substantially equal to the feeding flow rate of the biological composition.

In the same way, washing and elution steps are performed by a continuous and simultaneous feed and bleed. In this procedure the adsorption is resembling classical packed bed procedures and a significant number of theoretical plates are achieved and thereby a more efficient protein adsorption is taking place. This is in sharp contrast to the classical one-step batch adsorption procedure wherein only one equilibrium is taking place.

However, for some applications, one equilibrium stage may be enough for the specific purpose and in this case the mixing of the protein sorption material and the biological composition may be performed in one instant mixing step, while the subsequent washing and elution steps is performed as continuous and simultaneous feed and bleed.

The surface area of the filter relative to the adsorbent volume is influencing the productivity of the apparatus in terms of the amount of biomolecule that may be refined per litre of adsorbent per hour. If the filter area/adsorbent volume ratio is relatively low the productivity will be low, while, if the ratio is relatively high the productivity may be at a maximum.

Thus, in an embodiment of the present invention relating to the apparatus as described herein, the filter area relative to the volume of adsorbent is at least 0.01 $m^2$/L, such as at least 0.02 $m^2$/L, such as at least 0.05 $m^2$/L, such as at least 0.08 $m^2$/L, such as at least 0.10 $m^2$/L, such as at least 0.15 $m^2$/L, such as at least 0.20 $m^2$/L.

However, for some applications the filter area relative to the adsorbent volume may also become too large (e.g. in terms of equipment and maintenance cost for the filter unit and the total system volume), and it is therefore an embodiment of the invention that the filter area relative to the adsorbent volume is in the range of 0.01-0.5 $m^2$/L, such as 0.01-0.4 $m^2$/L, such as 0.01-0.3 $m^2$/L, such as 0.01-0.2 $m^2$/L, or such as in the range of 0.02-0.4 $m^2$/L, such as 0.02-0.3 $m^2$/L, such as 0.02-0.2 $m^2$/L, or such as in the range of 0.03-0.4 $m^2$/L, such as 0.03-0.3 $m^2$/L, such as 0.03-0.2 $m^2$/L, or such as in the range of 0.04-0.4 $m^2$/L, such as 0.04-0.3 $m^2$/L, such as 0.04-0.2 $m^2$/L, or such as in the range of 0.05-0.4 $m^2$/L, such as 0.05-0.3 $m^2$/L, such as 0.05-0.2 $m^2$/L, such as 0.05-0.15 $m^2$/L, such as 0.05-0.10 $m^2$/L.

*SPECIFIC EMBODIMENTS OF SORBENT MATERIAL*

An embodiment of the present invention relates to the apparatus as described herein, wherein the sorbent material is a cross-linked natural polysaccharide with a particle size distribution such that 90 % by volume of the particles has a diameter in the range of 10-1000 $\mu$m; and the ligand concentration is in the range of 1-300 millimoles/L; and the ligand is selected from the group consisting of ion exchange ligands, hydrophobic ligands, affinity sorption ligands, and mixed mode ligands; and the concentration of natural polysaccharide in the fully hydrated sorption material is in the range of 2-20 w% on a dry matter basis; and the natural cross-linked polysaccharide is selected from the group consisting of agar-agar, agarose, dextran, cellulose, and composites thereof; and the cross-linking is performed with reagents selected from the group consisting of epichlorohydrin, butanediol diglycidylether, allyl bromide, and allyl glycidylether; and the sorption material is in the form of particles.

Another embodiment of the present invention relates to the apparatus as described herein, wherein the sorbent material is a cross-linked natural polysaccharide with a particle size distribution such that 90 % by volume of the particles has a diameter in the range of 30-300 $\mu$m; and the ligand is selected from the group consisting of ion exchange ligands, affinity sorption ligands, and mixed mode ligands; and the concentration of natural polysaccharide in the fully hydrated sorption material is in the range of 3-12 w% on a dry matter basis; and the natural cross-linked polysaccharide is selected from the group consisting of agar-agar, agarose, cellulose, and composites thereof; and the cross-linking is performed with reagents selected from the group consisting of epichlorohydrin, butanediol diglycidylether, allyl bromide, and allyl glycidylether; and the sorption material is in the form of particles.

Yet another embodiment of the present invention relates to the apparatus as described herein, wherein the sorbent material is a cross-linked synthetic polymer with a particle size distribution such that 90 % by volume of the particles has a diameter in the range of 30-300 $\mu$m; and the ligand is selected from the group consisting of ion exchange ligands, affinity sorption ligands, and mixed mode ligands; and the concentration of synthetic polymer in the fully hydrated sorption material is in the range of 4-25 w% on a dry matter basis; and the synthetic cross-linked polymer is synthetic polymer is selected from the group consisting of polyacrylamide, polyacrylate, poly(allylamine), poly(allylalcohol), poly(vinylamine), poly(vinylalcohol), and composites thereof; and the cross-linking is performed with reagents selected from the group consisting of diacrylates, bis-acrylamides, allyl bromide, allyl glycidylether, and boric acid and mixtures and derivatives thereof; and the sorption material is in the form of particles.

**ADD-ONS**

The apparatus of the present invention may be constructed to comprise additional parts, for example: additional mixing tanks, inlets, outlets, permeate collection tanks, valves, and pumps, etc. Thus, an embodiment of the present invention relates to the apparatus as described herein, wherein the apparatus comprises one or more mixing tanks. However, a mixing tank is not essential for the apparatus of the invention and the mixing of the biological composition and the liquid comprising sorption material may be in the first fluid connections 3. Another embodiment of the present invention relates to the apparatus as described herein, wherein the apparatus comprises one or more first inlets. Yet another embodiment of the present invention relates to the apparatus as described herein, wherein the apparatus comprises one or more first outlets. Still another embodiment of the present invention relates to the apparatus as described herein, wherein the apparatus comprises one or more permeate collection tanks.

An embodiment of the present invention relates to the apparatus as described herein, wherein the second fluid connection 8 is equipped with a heat exchanger 14 as shown in figure 6. The heat exchanger makes it possible adjusting and controlling the temperature in the circulating flow. Control of the temperature may be important for obtaining a good viscosity of the mixture and/or an increased attachment of the target protein or other biomolecule to the sorption material.

The third fluid connection 9 is in some embodiments of the present invention e.g. as shown in figure 6, directed by further valves such that all permeate is returned to the mixing tank 2 through a sixth fluid connection 13 whereby the system volume should remain constant.

## WASHING/ELUTION/REGENERATION

An embodiment of the present invention relates to the apparatus as described herein, wherein the apparatus comprises one or more third inlets for adding a washing liquid. Alternatively or in addition, the apparatus may comprise washing tanks for easy addition of the washing liquid and for reducing risk of contamination. An embodiment of the present invention therefore relates to the apparatus as described herein, wherein the apparatus comprises one or more washing tanks. In this regard, the apparatus may also comprise outlets for draining the washing liquid. Thus, an embodiment of the present invention therefore relates to the apparatus as described herein, wherein the apparatus comprises one or more third outlets to drain the washing liquid from the apparatus.

Elution of the target protein or other biomolecule from the sorption material may be done by adding an elution liquid by use of a system installed for the purpose. An embodiment of the present invention therefore relates to the apparatus as described herein, wherein the apparatus comprises one or more fourth inlets for adding an elution liquid. Also, a further embodiment of the present invention relates to the apparatus as described herein, wherein the apparatus comprises one or more elution tanks. The elution tanks may be installed for easy addition of the elution liquid and for reducing risk of contamination. One other embodiment of the present invention relates to the apparatus as described herein, wherein the apparatus comprises one or more fourth outlets to drain the elution liquid from the apparatus.

The apparatus of the present invention is designed such that the sorption material may be used for a very long time without replacement or regeneration in an external regeneration system. Thus, the apparatus of the present invention may comprise regeneration tanks for easy addition of a regeneration liquid and for reducing any risk of contamination. An embodiment of the present invention therefore relates to the apparatus as described herein, wherein the apparatus comprises one or more regeneration tanks. In another embodiment of the present invention relating to the apparatus as described herein, the apparatus comprises one or more second inlets for adding a regeneration liquid. During or after regeneration of the sorption material the regeneration liquid may be drained by use of outlets installed for the purpose. Thus, an embodiment of the present invention relates to the apparatus as described herein, wherein the apparatus comprises one or more second outlets to drain the regeneration liquid from the apparatus.

It is to be understood that the first, second, third and fourth inlets and outlets may be the same inlets or outlets. Alternatively, one or more of the first, second, third or fourth inlets or outlets are individual inlet or outlets.

*WASHING/ELUTION/REGENERATION LIQUID*

The washing liquid may be any liquid which does not degrade or otherwise disturb the biomolecule-sorption material conjugate. That said, for most applications the washing liquid should be based on water. Thus, an embodiment of the present invention relates to the apparatus as described herein, wherein the washing liquid is water or an aqueous solution or an aqueous buffer solution. In this regard, an embodiment of the present invention relates to the apparatus as described herein, wherein the washing liquid comprises an inorganic acid or a salt thereof, or organic acid or a salt thereof, or mixtures of these. A specific embodiment of the present invention relates to the apparatus as described herein, wherein the washing liquid comprises an acid and/or its salt comprising sodium or potassium. Another specific embodiment of the present invention relates to the apparatus as described herein, wherein the washing liquid comprises one or more acids or salts thereof selected from the group consisting of hydrochloric acid, phosphoric acid, sulphuric acid, acetic acid, citric acid, caprylic acid, lactic acid and combinations thereof. An alternative embodiment of the present invention relates to the apparatus as described herein, wherein the washing liquid comprises a detergent. However, a most particular embodiment of the present invention relates to the apparatus as described herein, wherein the washing liquid is food grade.

Which elution liquid to utilize depends on the type of sorption material used for each specific application (i.e. specific type of target protein or other biomolecule to be refined). Thus, in a first example, separation of a particular target protein or other biomolecule involves a washing liquid and an elution liquid. However, in a second example, wherein another biomolecule is targeted, the washing liquid of the first example may be used as the elution liquid whereas the elution liquid of the first example may be used as the washing liquid. An embodiment of the present invention therefore relates to the apparatus as described herein, wherein the elution liquid is water or an aqueous salt solution or an aqueous buffer solution. In this regard, an embodiment of the present invention relates to the apparatus as described herein, wherein the elution liquid comprises an inorganic acid or a salt thereof or organic an acid or a salt thereof or mixtures of these. A specific embodiment of the present invention relates to the apparatus as described herein, wherein the elution liquid comprises an acid and/or its salt comprising sodium or potassium. Another specific embodiment of the present invention relates to the apparatus as described herein, wherein the elution liquid comprises one or more acids and/or their salts selected from the group consisting of hydrochloric acid, phosphoric acid, sulphuric acid, acetic acid, citric acid, caprylic acid, lactic acid and combinations thereof. An alternative embodiment of the present invention relates to the apparatus as described herein, wherein the elution liquid comprises a detergent. However, a most particular embodiment of the present invention relates to the apparatus as described herein, wherein the elution liquid is food grade.

Without being restricted to any particular liquids for use in regeneration of the sorption material, a particular embodiment of the present invention relates to the apparatus as described herein, wherein the regeneration liquid is a basic solution having a pH-value in the range of 8 to 14, such as 10 to 14, such as 12 to 13.

**THE PROCESS**

A second aspect of the present invention relates to a process for separation of a protein or other biomolecule comprised in a biological composition, the process comprising the steps of:

> i) mixing a biological composition comprising a target protein or other biomolecule with a composition comprising a sorption material for protein or other biomolecule to obtain a mixture, wherein the target protein or other biomolecule is carried by the sorption material,
>
> ii) filtrating the mixture using a tangential flow filtration unit having a membrane with nominal pore sizes in the range of 20 to 200 $\mu$m, such that at least 60% of particles larger than a value within 20 to 300 $\mu$m are retained by the filter having an average pore size of that value, for obtaining a first retentate comprising the target protein or other biomolecule and a first permeate which is optionally collected in a waste tank or a permeate collection tank,
>
> iii) recirculating and filtrating the first retentate by returning the obtained first retentate to the tangential flow filtration unit and thereby obtain a second permeate which is optionally collected, and obtaining a second retentate, and wherein the recirculation and further filtration in step iii) is carried out one or more times,
>
> iv) adding an elution liquid to the second retentate to release the target protein or other biomolecule from the sorption material,
>
> v) filtrating the second retentate using the tangential flow filtration unit to obtain a third retentate comprising the sorption material and a third permeate comprising the target protein or other biomolecule;
>
> vi) collecting the third permeate as a separated product composition comprising protein or other biomolecule, and wherein a pressure difference between the retentate side and permeate side of the filter in the tangential flow filtration unit is equalized or reversed resulting in a pulsed pressure increase and/or backward flush of the collected first permeate is obtained, said pressure difference is introduced at least once during the process during one or more of the steps ii) to iv), and wherein the pulsed pressure increase and/or backward flush is performed with a frequency in the range of 1-500 per hour and/or a pulse duration in the range of 0.01 to 200 seconds.

Another particular embodiment of the present invention relates to the process as described herein, wherein the first and second permeate is collected as a product composition.

An embodiment of the present invention relates to the process as described herein, wherein the process is performed using the apparatus as described herein.

Another embodiment of the present invention relates to the process as described herein, wherein the process comprises a step for washing the first and second retentate by adding a washing liquid.

A particular embodiment of the present invention relates to the process as described herein, wherein the frequent events are automatic and controlled by one or more electronic control units. Yet another embodiment of the present invention relates to the process as described herein, wherein the pulsed pressure increase and/or backward flush events are performed with a frequency in the range of 2-400 per hour, such as in the range of 2-300 per hour, such as in the range of 2-200 per hour, such as in the range of 2-150 per hour, such as in the range of 2-100 per hour, such as in the range of 2-80 per hour, such as in the range of 2-60 per hour, such as in the range of 3-60 per hour, such as in the range of 4-60 per hour, such as in the range of 5-60 per hour, such as in the range of 6-60 per hour, such as in the range of 6-50 per hour, such as in the range of 6-40 per hour, such as in the range of 6-30 per hour. Also, another embodiment of the present invention relates to the process as described herein, wherein the pulsed pressure increase and/or backward flush has a pulse duration is in the range of 0.05 to 150 seconds, such as in the range of 0.1 to 120 seconds, such as in the range of 0.2 to 100 seconds, such as in the range of 0.3 to 80 seconds, such as in the range of 0.3 to 60 seconds, such as in the range of 0.4 to 50 seconds, such as in the range of 0.5 to 40 seconds, such as in the range of 0.5 to 30 seconds, such as in the range of 0.5 to 25 seconds, such as in the range of 0.5 to 20 seconds, such as in the range of 0.5 to 15 seconds, such as in the range of 0.5 to 10 seconds, such as in the range of 0.5 to 8 seconds, such as in the range of 0.5 to 6 seconds, such as in the range of 0.5 to 5 seconds, such as in the range of 1.0 to 5 seconds.

A further embodiment of the present invention relates to the process as described herein, wherein the process comprises a step for regeneration of the sorption material by adding a regeneration liquid to the third retentate.

## ALTERNATIVE ASPECTS

A first alternative embodiment of the present invention relates to an apparatus comprising a first inlet 1 to a mixing tank 2 containing a sorption material suspended in an aqueous liquid at a concentration of 5-70 % by volume, said mixing tank having one or more first fluid connections 3 to a first end 4 of a tangential flow filtration unit 5 comprising a filter 6 with a nominal pore size in the range of 20 to 300 $\mu$m, and where a second end 7 of the tangential flow filtration unit 5 has a second fluid connection 8 configured to lead retentate back to the mixing tank 2; the tangential flow filtration unit 5 having a third fluid connection 9 configured to lead permeate to a first outlet 10; and wherein the apparatus comprises an automatic backward flush system being configured to apply a pulsed pressure increase and/or backward flush of the permeate with a frequency in the range of 1-500 per hour.

A second alternative embodiment of the present invention relates to an apparatus comprising a first inlet 1 to a mixing tank 2 having one or more first fluid connections 3 to a first end 4 of a tangential flow filtration unit 5 comprising a filter 6 with a nominal pore size in the range of 20 to 300 $\mu$m, and where a second end 7 of the tangential flow filtration unit 5 has a second fluid connection 8 configured to lead retentate back to the mixing tank 2; the tangential flow filtration unit 5 having a third fluid connection 9 configured to lead permeate to a first outlet 10; and wherein the apparatus comprises an automatic backward flush system being configured to apply a pulsed pressure increase and/or backward flush of the permeate with a frequency in the range of 1-500 per hour, and wherein the automatic backward flush system comprises:

- an opening-closing valve 19 in the third fluid connection 9 and which is being controlled by an electronic control unit 18,
- a first variable flow control valve 20 in the second fluid connection 8 and a second variable flow control valve 21 in the third fluid connection 9, and that are being controlled by an electronic control unit 18,
- a fourth fluid connection 11 configured to lead the permeate from the first outlet 10 to a permeate collection tank 15,
- a fifth fluid connection 12 connecting the permeate collection tank 15 to the permeate side of the filter 6,
- a centrifugal pump 16 inserted in the fifth fluid connection 12 and which centrifugal pump is also controlled by the electronic control units 18.

A third alternative embodiment of the present invention relates to an apparatus comprising a first inlet 1 to a mixing tank 2 containing a sorption material suspended in an aqueous liquid at a concentration of 5-70 % by volume, said mixing tank having one or more first fluid connections 3 to a first end 4 of a tangential flow filtration unit 5 comprising a filter 6 with a nominal pore size in the range of 20 to 300 $\mu$m, and where a second end 7 of the tangential flow filtration unit 5 has a second fluid connection 8 configured to lead retentate back to the mixing tank 2; the tangential flow filtration unit 5 having a third fluid connection 9 configured to lead permeate to a first outlet 10; and wherein one or more of the first fluid connections 3 and/or the second fluid connection 8 comprise one or more peristaltic pumps 17 configured to exert a low shear force to a protein sorption material when in use, and wherein the apparatus comprises an automatic backward flush system being configured to apply a pulsed pressure increase and/or backward flush of the permeate with a frequency in the range of 1-500 per hour, and wherein the automatic backward flush system comprises:

- an opening-closing valve 19 in the third fluid connection 9 and which is being controlled by an electronic control unit 18,
- a first variable flow control valve 20 in the second fluid connection 8 and a second variable flow control valve 21 in the third fluid connection 9, and that are being controlled by an electronic control unit 18,
- a fourth fluid connection 11 configured to lead the permeate from the first outlet 10 to a permeate collection tank 15,
- a fifth fluid connection 12 connecting the permeate collection tank 15 to the permeate side of the filter 6,
- a centrifugal pump 16 inserted in the fifth fluid connection 12 and which centrifugal pump is also controlled by the electronic control units 18.

A fourth alternative embodiment of the present invention relates to an apparatus comprising a first inlet 1 to a mixing tank 2 having one or more first fluid connections 3 to a first end 4 of a tangential flow filtration unit 5 comprising a filter 6 with a nominal pore size in the range of 20 to 300 $\mu$m, and where a second end 7 of the tangential flow filtration unit 5 has a second fluid connection 8 configured to lead retentate back to the mixing tank 2; the tangential flow filtration unit 5 having a third fluid connection 9 configured to lead permeate to a first outlet 10; and wherein one or more of the first fluid connections 3 and/or the second fluid connection 8 comprise one or more peristaltic pumps 17 configured to exert a low shear force to a protein sorption material when in use, and wherein the apparatus comprises an automatic backward flush system being configured to apply a pulsed pressure increase and/or backward flush of the permeate with a frequency in the range of 1-500 per hour, and wherein the automatic backward flush system comprises:

- an opening-closing valve 19 in the third fluid connection 9 and which is being controlled by an electronic control unit 18,

- a first variable flow control valve 20 in the second fluid connection 8 and a second variable flow control valve 21 in the third fluid connection 9, and that are being controlled by an electronic control unit 18,
- a fourth fluid connection 11 configured to lead the permeate from the first outlet 10 to a permeate collection tank 15,
- a fifth fluid connection 12 connecting the permeate collection tank 15 to the permeate side of the filter 6,
- a centrifugal pump 16 inserted in the fifth fluid connection 12 and which centrifugal pump is also controlled by the electronic control units 18.

A fifth alternative embodiment of the present invention relates to an apparatus comprising a first inlet 1 to a mixing tank 2 containing a sorption material suspended in an aqueous liquid at a concentration of 5-70 % by volume, said mixing tank 2 having one or more first fluid connections 3 to a first end 4 of a tangential flow filtration unit 5 comprising a filter 6 with a nominal pore size in the range of 20 to 300 $\mu$m, and where a second end 7 of the tangential flow filtration unit 5 has a second fluid connection 8 configured to lead retentate back to the mixing tank 2; the tangential flow filtration unit 5 having a third fluid connection 9 configured to lead permeate to a first outlet 10; and wherein the apparatus comprises an automatic backward flush system being configured to apply a pulsed pressure increase and/or backward flush of the permeate with a frequency in the range of 1-500 per hour and/or a pulse duration of between 0.01 to 200 seconds.

It should be noted that embodiments and features described in the context of one of the aspects of the present invention also apply to the other aspects of the invention.

The invention will now be described in further details in the following non-limiting examples.

## Examples

### *Example 1* - *Test of apparatus and backward flush*

Figure 6 shows a schematic representations of an apparatus used for the examples. The mixing tank 2 was a cylindrical stainless steel tank with a conical bottom and the first fluid connection 3 comprised a variable flow peristaltic tube pump 17 having a maximum flow capacity of 75 L/min. The tangential flow filtration unit 5 was a cylindrical unit equipped with a filter tube made of melt blown polypropylene and having a nominal pore size of 30 micron. The filter tube had a length of 1200 mm, an outer diameter of 30 mm and an inner diameter of 20 mm. The total inner surface filter area of the filter tube was 0.076 m$^2$.

The permeate third fluid connection 9 was connected to a first outlet 10 that would either direct the permeate to a waste tank for collection or through a fourth fluid connection 11 for collection in a permeate collection tank 15. The permeate third fluid connection 9 was further equipped with an opening-closing valve 19 and an electronic control unit 18 (back-pulse controller) to switch the valve position with a chosen frequency. The same controller 18 was controlling a centrifugal pump 16 connected to a permeate back flush port on the tangential flow filtration unit 5 through a fifth fluid connection 12, which pump 16, when activated, reverses the flow of permeate fluid to obtain a pulsed pressure increase/backward flush through the filter tubes. Thus, when the pump 16 was activated, the opening-closing valve 19 at the third fluid connection 9 were closed and liquid delivered by the centrifugal pump 16 then entered the permeate side of the filter unit through the fifth fluid connection 12. The centrifugal pump 16 was connected to the permeate tank 15 and the pulsed pressure increase/backward flush was performed with permeate collected prior to applying the pulsed pressure increase/backward flush.

The apparatus was further equipped with monitors to monitor flow rates, temperature and pressure in all fluid connections. Variable flow control valves 20,21 positioned in the second and third fluid connections 8,9 were used to regulate the flow and thereby also the back pressure drop across the length of the tangential filtration unit 5 and across the filter wall (from retentate to permeate side) in combination with regulation of the flow rate of the variable peristaltic pump 17.

The permeate outlet was directed by a valve such that all permeate was returned to the mixing tank 2 through a sixth fluid connection 13, whereby the system volume remained constant throughout the test.

For testing of the apparatus with water and adsorbent the mixing tank was filled with 20 L of an aqueous suspension of 6 % agarose beads with a particle size in the range of 45-165 micron (Cytiva, USA). The concentration of cross-linked agarose beads in the suspension was 31 %, as determined by centrifugation of a sample of the suspension for 5 minutes at 3000 G (i.e. a 100 ml sample would have 31 ml sedimented beads after centrifugation). The system volume at the outset of the test was thus 20 L.

### *Test A. Flux rates with and without backward flush*

For this test the peristaltic pump was set to a flow rate of 20 L/min whereby the adsorbent suspension was recirculated through the first fluid connection to pass the tangential flow filtration unit and returning to the mixing tank through the second fluid connection. At the outset of the test, the permeate third and fifth fluid connections were fully closed such that no permeate could pass the filtration unit. Under these conditions, the pressure in the first fluid connection measured at the first end of the tangential flow filtration unit was 0.1 bar and the pressure of the second fluid connection measured at the second end of the filtration unit was 0 bar. The pressure gradient between the retentate side of the filters and the permeate side of the filters (TMP) was defined as follows:

$$TMP = (feed\ pressure + retentate\ pressure) / 2 - permeate\ pressure$$

By opening the second variable flow control valve and applying back pressure by adjusting the first variable flow control valve on the return second fluid connection, TMP was adjusted to set values. For this test, TMP was successively set to the following values in three individual runs: 0.225 Bar, 0.400 Bar, and 0.800 Bar.

Once the second variable flow control valve was opened and TMP was set to the desired value, a pulsed pressure increase and/or backward flush (BP) i.e. back pressure cycle was started where the opening-closing valve was closed and the centrifugal pump was activated to reverse permeate flow through the fifth fluid connection. The pump was activated for 3 seconds and then the pump was stopped and the opening-closing valve opened again. This cycle was repeated once every minute and the permeate flow was measured 10s after every BP. Once the system had run for 11 minutes, the BP cycle was shut off for a period as indicated below, while the permeate flow measurement was continued in intervals during this period.

- With TMP at 0.225 Bar, the system was allowed to run without BP for 32 min.
- With TMP at 0.400 Bar, the system was allowed to run without BP for 16 min.
- With TMP at 0.800 Bar, the system was allowed to run without BP for 10 min.

Following the indicated time periods without BP, the BP cycle was reengaged and permeate flow was again measured 10s after every BP.

The flux, defined as litre permeate per hour per square meter filter area was calculated from each permeate flow measurement. Thus, for a membrane area of 0.076 m$^2$ a permeate flow of 1 L/min is equal to a flux of 1 x 60 x (1/0.076) = 789.5 L/m$^2$/h, or 789.5 LMH.

Figure 7 shows that when the apparatus operates with frequent backward flush events activated, then the flux is above 3300 LMH and increasing with TMP up to about 0.4 bar, but when the BP cycle is stopped flux decreases very fast to approx. 1500 LMH for all TMP's tested. This flux inhibition worsens over time, however once the BP cycle is reengaged, flux returns to the initial levels nearly instantaneously.

Conclusion: The backward flux system was effective at ensuring a high flux through the filter.

### Example 2 - Preparation of epichlorohydrin activated agarose beads.

6 % agarose beads having a particle size range of 45-160 micron (Cytiva, USA) were reacted with epichlorohydrin as follows: 20 L beads were suspended in 16 L water in a closed reaction vessel with stirring. The suspension was heated to 40 °C and then added 2.4 L epichlorohydrin (Sigma-Aldrich, USA) and 2.0 L 32.5 % (w/w) sodium hydroxide. The suspension was stirred and maintained at 40 °C for 3 hours, then it was transferred to a nutsche filter and washed with 200 L water. The beads where then drained for interstitial water whereby the activated beads could be removed as a filter cake. The washed and drained beads had a content of 52 micromole epoxy-groups per gram drained beads.

### Example 3 -Preparation of a protein adsorbent having benzylamine ligands attached.

20 L epichlorohydrin activated beads as prepared in example 2, were suspended in 16 L water and transferred to a closed reaction vessel with stirring. The suspension was then added 2 L benzylamine (Sigma-Aldrich, USA) and the pH of the suspension was adjusted to 10.5 with hydrochloric acid. The suspension was stirred at ambient temperature for 24 hours and then it was transferred to a nutsche filter and washed sequentially with 50 L water, 200 L 0.05 M hydrochloric acid and 100 L water. The beads where then drained for interstitial water, whereby the benzylamine coupled beads could be removed as a filter cake. The washed and drained beads had a content of 42 micromole benzylamine-groups per gram drained beads.

### Example 4 -Preparation of a sulfonic acid ion exchange adsorbent.

An ion exchange adsorbent was prepared by cross-linking 6 % agarose beads with particle sizes in the range of 45-160 micron (Cytiva, USA). The beads were reacted with epichlorohydrin as follows: 20 L beads were suspended in 15 L 0.2 M sodium sulphate (Sigma Aldrich, USA) in a closed reaction vessel with stirring. The suspension was heated to 40 °C and then added 1.2 L epichlorohydrin (Sigma-Aldrich, USA) and 1.5 L 32.5 % (w/w) sodium hydroxide. The suspension was stirred and maintained at 40 °C for 18 hours and then it was transferred to a filter nutsche filter and washed with 200 L water. The beads where then drained for interstitial water whereby the activated beads could be removed as a filter cake.

Reaction with sulfonating reagent: The cross-linked beads were then suspended in 20 L 32.5 % w/w sodium hydroxide and transferred back to the reaction vessel as a suspension. The suspension was added 0.8 kg sodium dodecyl sulphate and then heated to 65 °C under gentle stirring. Hereafter 10 kg 1,4-butane sultone (Organica, Germany) was added under continuous stirring in 10 x 1 kg aliquots with 30 minutes intervals. The temperature was regulated to remain within the range of 65-75 °C. Following the last addition of butane sultone the mixture was allowed to react for an additional 2 hours. After the reaction the suspension was transferred to a nutsche filter and washed sequentially with 200 L water, 200 L 0.05 M sodium hydroxide and 400 L water. The beads where then drained for interstitial water whereby the butane sultone coupled beads could be removed as a filter cake. The washed and drained beads were found to have a content of 185 micromole titratable sulfonic acid groups per gram drained beads.

*Example 5* -*Preparation of a quaternary amine ion exchange adsorbent.*

A quaternary amine ion exchange adsorbent was prepared by cross-linking of 6 % agarose beads having a particle size range of 45-160 micron (Cytiva, USA). The beads were reacted with epichlorohydrin as follows: 20 L beads were suspended in 15 L 0.2 M sodium sulphate (Sigma Aldrich, USA) in a closed reaction vessel with stirring. The suspension was heated to 40 °C and then added 1.2 L epichlorohydrin (Sigma-Aldrich, USA) and 1.5 L 32.5 % (w/w) sodium hydroxide. The suspension was stirred and maintained at 40 °C for 18 hours and then it was transferred to a nutsche filter and washed with 200 L water. The beads were then drained for interstitial water whereby the cross-linked beads could be removed as a filter cake.

Reaction with quaternary amine reagent: The cross-linked beads were then suspended in 15 L 0.6 M sodium hydroxide and transferred back to the reaction vessel as a suspension. The suspension was then added 994 g sodium sulphate and heated to 65 °C under gentle stirring. Hereafter 12 kg glycidyl-trimethyl-ammonium chloride (Sigma Aldrich, USA) was added under continuous stirring in 10 x 1 kg aliquots with 30 minutes intervals. The temperature was regulated to remain within the range of 55-65 °C. Following the last addition of the reagent the mixture was allowed to react for an additional 20 hours. After the reaction the suspension was transferred to a nutsche filter and washed sequentially with 200 L water, 200 L 0.05 M hydrochloric acid and 200 L water. The beads were then drained for interstitial water whereby the quaternary amine coupled beads could be removed as a filter cake. The washed and drained beads were found to have a content of 175 micromole quaternary amine groups per gram drained beads.

*Example 6* - *isolation of beta-lactoglobulin from bovine skim milk.*

This example demonstrates the use of the apparatus according to the invention, such as that illustrated in figure 6, for isolation of beta-lactoglobulin (beta-lg) selectively from bovine skim milk.

*Materials and Methods*

Adsorbent:

Quaternary amine ion exchange adsorbent, as described in example 5. The adsorbent was suspended in water at a concentration of 30 % as determined by centrifugation at 3000 G for 5 minutes. The suspension was filled into the mixing tank 2 and the system volume was 21 L which means a total volume of 6.3 L of adsorbent was employed.

Raw material:

Skim milk was sourced from a local dairy.

Equipment setup

(numbers are referring to figure 6):

*Tangential flow unit:*

The tangential flow filtration unit 5 was a cylindrical unit equipped with seven filter tubes made of melt blown polypropylene having nominal pore size of 30 micron.

The dimensions of the filter tubes were: outer diameter: 10 mm, inner diameter: 6 mm, and length: 300 mm. The filtration area for the filter in each filter tube was found to be: 56.5 cm$^2$ and thus the total filter area for the filter 6 was 395.6 cm$^2$ (0.04 m$^2$).

*System settings:*

The peristaltic pump 17 was set at 22 L/min. The trans membrane pressure (TMP) was set to be 0.2 bar during the entire experiment. TMP was regulated by adjusting the opening-closing 19 and the first variable flow control valve 20.

Backward flush setting: A backward flush was performed every 24 seconds during wash and elution of the adsorbent. The centrifugal pump 16 was running for 1 sec. during each backward flush. The backward flush was fed from the permeate collection tank 15, containing water. The backward flush system was not running during load of raw material. A separate peristaltic pump (not shown in figure 6) was used to feed raw material, wash and elution buffers respectively through the first inlet 1 into the mixing tank 2. The pump speed was regulated to be the same as the permeate flow through the third fluid connection 9 to keep a constant volume in the mixing tank 2. The permeate stream through the third fluid connection 9 was connected to separate tanks (not shown in figure 6) to collect respectively non-bound fraction, wash fraction and product fraction (eluate).

The chemicals used in the examples, e.g. for preparing buffers and solutions are commercial products of at least reagent grade. Water used for conducting the experiments was de-ionized water.

Buffer solutions

*0.25 M citric acid pH 4.0*
48 g of citric acid, from BDH Chemicals
Addition of 900 ml of water and pH is adjusted to 4.5 with 1 M NaOH. Water is then added to a finally volume of 1.0 L.
*1 M citric acid*
192 g of citric acid, from BDH Chemicals
Addition of water to 1.0 L.

SDS-PAGE electrophoresis reagents

a) LDS sample buffer, 4X is obtained from Abcam, UK
b) SDS Run buffer, 20x is obtained from Abcam, UK
c) Precast 16% gels are obtained from Abcam, UK
d) Instant Stain 15 min Coomassie blue for proteins was obtained from Kem-En-Tec Nordic, DK.

Assays

*a) SDS-PAGE electrophoresis:*

The samples produced in each example were analysed using SDS-PAGE gel electrophoresis showing the protein composition in each sample. The SDS-PAGE gel electrophoresis was performed using an electrophoresis apparatus and precast 16 gels from Abcam, UK. The protein samples were mixed with LDS sample buffer and incubated for 10 minutes at 70°C. The samples were applied to the precast gel and proteins were allowed to run for one hour at 200 V 90 mA in the SDS Run buffer at non-reduced running conditions. The gel was developed in the staining solution for 18 hours. The protein bands were evaluated by visually inspection or analysed by scanning densitometry to quantify the amount of specific proteins in the test solutions.

*b) Spectrophotometric measurement of permeate samples*

Spot samples were taken from the permeate stream (i.e. from the third fluid connection 9 on figure 6) during wash and elution. These were measured on a spectrophotometer (VWR, UV-3100PC) at 280 nm. These readings were used to determine efficiency of wash and elution of the adsorbent.

*Procedure*

The filtration system was started with settings as described in materials and methods. When TMP was regulated to 0.2 bar the permeate flow was 2.1 L/min, and at the same time skim milk was pumped into the mixing tank 2 with the same flow rate, maintaining a constant system volume and resulting in a residence time of 10 minutes.

Ten ml spot samples were withdrawn from the permeate stream in the third fluid connection 9 during load. After loading 49 L skim milk, washing with water was initiated and the backward flush system was engaged. An external feed pump was pumping water into the mixing tank 2 to wash out non-bound skim milk components at a flow rate of 3 L/min. In total 110 L of water was used for the washing step. Ten ml spot samples were withdrawn from the permeate stream in the third fluid connection 9 during the washing procedure.

Following washing, the elution of bound beta-lg was initiated: Initially the permeate opening-closing valve 19 was closed, and the external feed pump and the backward flush system were stopped. The retentate was then recirculated through the tangential flow filtration unit 5 without any permeate flow. To release bound proteins from the adsorbent 2.3 L of 0.25 M sodium citrate pH 4.0 was added to the mixing tank 2, to reach a final concentration of 25 mM sodium citrate, and pH was adjusted to pH 4.5 by addition of 1 M citric acid. After 10 minutes of recirculation the permeate the opening-closing valve 19 was opened again to reach a TMP of 0.2 bar. The resulting permeate flow was 3.0 L/min. At first, 2.3 L of permeate was collected without the external feed pump started to reach a system volume of 21 L. Then the external feed pump was started to pump water into the mixing tank 2 to wash out the released protein at a flow rate of 3 L/min and with the backward flush system being active. During elution, 10 ml spot samples were taken from the permeate stream in the third fluid connection 9. In total 80 L of water was used to wash/elute out the desorbed beta-lactoglobulin product.

All the collected spot samples were loaded on SDS-PAGE gels. The spot samples from wash and elution were also analysed by spectrophotometry at 280 nm.

*Results*

Spectrophotometric results:

Figure 8 shows values of optical density (OD) obtained at 280 nm for the spot samples as a function of system volumes of water pumped into the mixing tank during wash and elution (i.e. 1 system volume = 21 L).

The graph in figure 8 illustrates that the permeate absorbance decreases during washing. After washing with 5.25 system volumes of water less than 10 % of the initial OD reading (measured at 280 nm) is left in the permeate. At the arrow the elution starts and the OD reading increases due to release of the beta-lactoglobulin. Eluting with 3.8 system volumes of water reduces the OD reading in the permeate with 85 %. Thus, about 190 L of water was used for the total process of obtaining a permeate comprising the refined protein and for which the OD reading was decreased by 85% when compared to the initial composition.

The average permeate flow during load of skim milk was 2.1 L/min which corresponds to a flux of 3150 LMH and the average flow during washing and elution was 3.0 L/min which corresponded to a flux of 4500 LMH.

SDS-PAGE results:

*Initial binding of beta-lg to the protein sorption material:*

Protein binding efficiency and capacity of the adsorbent was investigated by SDS-PAGE analysis of the permeate obtained during filtration of the mixture comprising beta-lg on the adsorbent carrier. The SDS-PAGE results are shown in figure 9 with each of the lanes representing:

Lane 1: Skim milk
Lane 2: Spot sample of permeate after load of 5 L skim milk to the mixing tank,
Lane 3: Spot sample of permeate after load of 10 L skim milk to the mixing tank,
Lane 4: Spot sample of permeate after load of 15 L skim milk to the mixing tank,
Lane 5: Spot sample of permeate after load of 20 L skim milk to the mixing tank,
Lane 6: Spot sample of permeate after load of 25 L skim milk to the mixing tank,
Lane 7: Spot sample of permeate after load of 30 L skim milk to the mixing tank,
Lane 8: Spot sample of permeate after load of 35 L skim milk to the mixing tank,
Lane 9: Spot sample of permeate after load of 40 L skim milk to the mixing tank,
Lane 10: Spot sample of permeate after load of 42 L skim milk to the mixing tank.

From the SDS-PAGE analysis depicted in figure 9 it was concluded that the anion exchange adsorbent binds almost all the beta-lactoglobulin present in the skim milk. After loading of 30 L of skim milk (lane 7-10) only a very small amount of beta-lactoglobulin starts to appear in the permeate. It is estimated to be less than 5 % of the total applied beta-lactoglobulin.

*Wash of beta-lg bound to the protein sorption material:*

The ability of the protein sorption material to prevent elution of beta-lg during application of the washing liquid (i.e. water) was investigated by SDS-PAGE analysis of the permeate obtained during washing. These SDS-PAGE results are shown in figure 10 with each of the lanes representing:

Lane 1: Spot sample of permeate after wash with 0.95 system volume,
Lane 2: Spot sample of permeate after wash with 1.9 system volumes,
Lane 3: Spot sample of permeate after wash with 2.86 system volumes,
Lane 4: Spot sample of permeate after wash with 3.81 system volumes,
Lane 5: Spot sample of permeate after wash with 4.53 system volumes,
Lane 6: Spot sample of permeate after wash with 5.24 system volumes.

From the SDS-PAGE analysis in figure 10 it was concluded that after washing with 5.24 system volumes of water the major part of the non-bound proteins, where casein is most dominant, are washed out.

*Elution and collection of the refined permeate comprising the target protein:*
Release of beta-lg from the sorbent material by adding citric acid elution solutions to adjust pH in the retentate flow to 4.5 was investigated by SDS-PAGE analysis of the permeate obtained during the elution step. The results are shown in figure 11 wherein each of the lanes represent:

Lane 1: Spot sample of permeate after elution with 0.71 system volume,
Lane 2: Spot sample of permeate after elution with 1.43 system volumes,
Lane 3: Spot sample of permeate after elution with 1.90 system volumes,
Lane 4: Spot sample of permeate after elution with 2.38 system volumes,
Lane 5: Spot sample of permeate after elution with 2.86 system volumes,
Lane 6: Spot sample of permeate after elution with 3.33 system volumes,
Lane 7: Spot sample of permeate after elution with 3.8 system volumes.

From the SDS-PAGE analysis in figure 11 can be seen that the eluate fractions contain highly purified beta-lactoglobulin permeate with only trace amounts of other proteins. The water washes out the eluted beta-lactoglobulin resulting in a decreasing concentration of the protein in the permeates going from lane 1 to 7 (see figure 11).

**Number listing**

1. First inlet.
2. Mixing tank.
3. First fluid connections.
4. First end (of the tangential flow filtration unit).
5. Tangential flow filtration unit.
6. Filter.
7. Second end (of the tangential flow filtration unit).
8. Second fluid connection.
9. Third fluid connection.
10. First outlet.
11. Fourth fluid connection.
12. Fifth fluid connection.
13. Sixth fluid connection.
14. Heat exchanger.
15. Permeate collection tank.
16. Centrifugal pump.
17. Peristaltic pump.
18. Electronic control units.

19. Opening-closing valve.
20. First variable flow control valve.
21. Second variable flow control valve.

**References**

- WO 2015/118223

**Claims**

1. An apparatus comprising a first inlet (1) and one or more first fluid connections (3) configured to lead fluid from the first inlet (1) to a first end (4) of a tangential flow filtration unit (5) comprising a filter (6) with a nominal pore size in the range of 20 to 300 $\mu$m, such that at least 60% of particles larger than a value within 20 to 300 $\mu$m are retained by the filter having an average pore size of that value, and wherein the first fluid connections (3) comprises a sorption material for protein or other biomolecule suspended in an aqueous liquid at a concentration of 5-70 % by volume, and where a second end (7) of the tangential flow filtration unit (5) has a second fluid connection (8) configured to lead retentate back to the first end of the tangential flow filtration unit (5); the tangential flow filtration unit (5) having a third fluid connection (9) configured to lead permeate to a first outlet (10); and wherein the apparatus comprises an automatic backward flush system being configured to apply a pulsed pressure increase and/or backward flush of the permeate with a frequency in the range of 1-500 per hour and/or a pulse duration of between 0.01 to 200 seconds.

2. The apparatus according to claim 1 wherein the automatic backward flush system comprises:

    - an opening-closing valve (19) in the third fluid connection (9) being controlled by an electronic control unit (18).

3. The apparatus according to any one of the preceding claims, wherein the automatic backward flush system comprises:

    - a first variable flow control valve (20) in the second fluid connection (8) and a second variable flow control valve 21 in the third fluid connection (9), that are being controlled by an electronic control unit (18).

4. The apparatus according to any one of the preceding claims, wherein the automatic backward flush system comprises:

    - a fourth fluid connection (11) configured to lead the permeate from the first outlet 10 to a permeate collection tank (15),
    - a fifth fluid connection (12) connecting the permeate collection tank (15) to the permeate side of the filter (6), and
    - a centrifugal pump (16) inserted in the fifth fluid connection (12) and which centrifugal pump is also controlled by the electronic control units (18).

5. The apparatus according to any one of the preceding claims, wherein a mixing tank is connected between the first inlet (1) and the first fluid connections (3), and wherein the mixing tank comprises the sorption material.

6. The apparatus according to any one of the preceding claims, wherein the filter (6) is a high flux filter with a clean water permeate flux of more than 1000 L/m$^2$/h when the TMP is about 0.1-0.9 bar.

7. The apparatus according to any one of the preceding claims, wherein the sorption material has a particle size distribution such that 90 % by volume of the particles has a diameter in the range of 10-1000 $\mu$m.

8. The apparatus according to any one of the preceding claims, wherein the sorption material is a polymer sorption material comprising one or more polymer selected from the group consisting of natural polysaccharide, agar-agar, agarose, dextran, cellulose, cellulose ethers, hydroxypropyl cellulose, carboxymethyl celluose, starches, gum, guar gum, gum arabic, gum ghatti, gum tragacanth, locust bean gum, xanthan gum, pectin, mucin, chitin, chitosan, alginate, carrageenan, heparin, gelatin, synthetic polymer, polyamide, polyacrylamide, polymethacrylamide, polyimide, polyacrylate, polymeric vinyl, polystyrene, polyalkene, polyvinyl alcohol, polyester, polyether, silicious material, silicon dioxide, amorphous silica, quartz, silica, metal silicate, controlled pore glass, ceramic, metal oxide, sulphide, and composites thereof.

9. A process for separation of a protein or other biomolecule comprised in a biological composition, the process comprising the steps of:

i) mixing a biological composition comprising a target protein or other biomolecule with a composition comprising a sorption material for protein or other biomolecule to obtain a mixture, wherein the target protein or other biomolecule is carried by the sorption material,

ii) filtrating the mixture using a tangential flow filtration unit having a membrane with nominal pore sizes in the range of 20 to 300 $\mu$m, such that at least 60% of particles larger than a value within 20 to 300 $\mu$m are retained by the filter having an average pore size of that value, for obtaining a first retentate comprising the target protein or other biomolecule and a first permeate which is optionally collected in a waste tank or a permeate collection tank,

iii) recirculating and filtrating the first retentate by returning the obtained first retentate to the tangential flow filtration unit and thereby obtain a second permeate which is optionally collected, and obtaining a second retentate, and wherein the recirculation and further filtration in step iii) is carried out one or more times,

iv) adding an elution liquid to the second retentate to release the target protein or other biomolecule from the sorption material,

v) filtrating the second retentate using the tangential flow filtration unit to obtain a third retentate comprising the sorption material and a third permeate comprising the target protein or another biomolecule;

vi) collecting the third permeate as a separated product composition comprising protein or other biomolecule, and wherein a pressure difference between the retentate side and permeate side of the filter in the tangential flow filtration unit is equalized or reversed resulting in a pulsed pressure increase and/or backward flush of the collected first permeate is obtained, said pressure difference is introduced at least once during the process during one or more of the steps ii) to iv), and wherein the pulsed pressure increase and/or backward flush is performed with a frequency in the range of 1-500 per hour and/or a pulse duration in the range of 0.01 to 200 seconds.

**Patentansprüche**

1. Vorrichtung, umfassend einen ersten Einlass (1) und eine oder mehrere erste Fluidverbindungen (3), die so konfiguriert sind, dass sie Fluid von dem ersten Einlass (1) zu einem ersten Ende (4) einer Tangentialströmungs-Filtrationseinheit (5) leiten, die einen Filter (6) mit einer nominalen Porengröße in dem Bereich von 20 bis 300 $\mu$m umfasst, sodass mindestens 60 % der Partikel, die größer sind als ein Wert innerhalb von 20 bis 300 $\mu$m, von dem Filter mit einer durchschnittlichen Porengröße dieses Wertes zurückgehalten werden, und wobei die ersten Fluidverbindungen (3) ein Sorptionsmaterial für Proteine oder andere Biomoleküle umfassen, das in einer wässrigen Flüssigkeit in einer Konzentration von 5-70 Vol.-% suspendiert ist, und wobei ein zweites Ende (7) der Tangentialströmungs-Filtrationseinheit (5) eine zweite Fluidverbindung (8) aufweist, die so konfiguriert ist, dass sie das Retentat zurück zu dem ersten Ende der Tangentialströmungs-Filtrationseinheit (5) leitet; wobei die Tangentialströmungs-Filtrationseinheit (5) eine dritte Fluidverbindung (9) aufweist, die so konfiguriert ist, dass sie Permeat zu einem ersten Auslass (10) leitet; und wobei die Vorrichtung ein automatisches Rückspülsystem umfasst, das so konfiguriert ist, dass es einen gepulsten Druckanstieg und/oder eine Rückspülung des Permeats mit einer Frequenz in dem Bereich von 1 - 500 pro Stunde und/oder einer Impulsdauer zwischen 0,01 und 200 Sekunden anwendet.

2. Vorrichtung nach Anspruch 1, wobei das automatische Rückspülsystem umfasst:

- ein Öffnungs-Schließ-Ventil (19) in der dritten Fluidverbindung (9), das von einer elektronischen Steuereinheit (18) gesteuert wird.

3. Vorrichtung nach einem der vorstehenden Ansprüche, wobei das automatische Rückspülsystem umfasst:

- ein erstes variables Strömungsregelventil (20) in der zweiten Fluidverbindung (8) und ein zweites variables Strömungsregelventil (21) in der dritten Fluidverbindung (9), die von einer elektronischen Steuereinheit (18) gesteuert werden.

4. Vorrichtung nach einem der vorstehenden Ansprüche, wobei das automatische Rückspülsystem umfasst:

- eine vierte Fluidverbindung (11), die so konfiguriert ist, dass sie das Permeat von dem ersten Auslass (10) zu einem Permeatsammeltank (15) leitet,

- eine fünfte Fluidverbindung (12), die den Permeatsammeltank (15) mit der Permeatseite des Filters (6) verbindet, und

- eine Zentrifugalpumpe (16), die in die fünfte Fluidverbindung (12) eingesetzt ist und wobei die Zentrifugalpumpe ebenfalls von den elektronischen Steuereinheiten (18) gesteuert wird.

5. Vorrichtung nach einem der vorstehenden Ansprüche, wobei ein Mischbehälter zwischen dem ersten Einlass (1) und den ersten Fluidverbindungen (3) verbunden ist und wobei der Mischbehälter das Sorptionsmaterial umfasst.

6. Vorrichtung nach einem der vorstehenden Ansprüche, wobei der Filter (6) ein Hochdurchflussfilter mit einem Reinwasser-Permeat-Durchfluss von mehr als 1000 L/m$^2$/h ist, wenn der TMP etwa 0,1 - 0,9 Bar beträgt.

7. Vorrichtung nach einem der vorstehenden Ansprüche, wobei das Sorptionsmaterial eine Partikelgrößenverteilung aufweist, sodass 90 Vol.-% der Partikel einen Durchmesser in dem Bereich von 10 - 1000 $\mu$m aufweisen.

8. Vorrichtung nach einem der vorstehenden Ansprüche, wobei das Sorptionsmaterial ein polymeres Sorptionsmaterial ist, das ein oder mehrere Polymere umfasst, ausgewählt aus der Gruppe, bestehend aus natürlichem Polysaccharid, Agar-Agar, Agarose, Dextran, Cellulose, Celluloseethern, Hydroxypropylcellulose, Carboxymethylcellulose, Stärken, Gummi, Guargummi, Gummi arabicum, Ghatti-Gummi, Gummitragant, Johannisbrotkernmehl, Xanthangummi, Pektin, Mucin, Chitin, Chitosan, Alginat, Carrageenan, Heparin, Gelatine, synthetischem Polymer, Polyamid, Polyacrylamid, Polymethacrylamid, Polyimid, Polyacrylat, polymeres Vinyl, Polystyrol, Polyalken, Polyvinylalkohol, Polyester, Polyether, silikatisches Material, Siliciumdioxid, amorphes Siliciumdioxid, Quarz, Siliziumdioxid, Metallsilikat, porenkontrolliertes Glas, Keramik, Metalloxid, Sulfid und deren Verbundstoffe.

9. Prozess zur Abtrennung eines Proteins oder eines anderen Biomoleküls, das in einer biologischen Zusammensetzung umfasst ist, wobei der Prozess die Schritte umfasst von:

i) Mischen einer biologischen Zusammensetzung, die ein Zielprotein oder ein anderes Biomolekül umfasst, mit einer Zusammensetzung, die ein Sorptionsmaterial für ein Protein oder ein anderes Biomolekül umfasst, um eine Mischung zu erhalten, wobei das Zielprotein oder das andere Biomolekül von dem Sorptionsmaterial getragen wird,

ii) Filtrieren der Mischung unter Verwendung einer Tangentialströmungs-Filtrationseinheit, die eine Membran mit Nennporengrößen in dem Bereich von 20 bis 300 $\mu$m aufweist, sodass mindestens 60 % der Partikel, die größer sind als ein Wert innerhalb von 20 bis 300 $\mu$m, von dem Filter mit einer durchschnittlichen Porengröße dieses Wertes zurückgehalten werden, um ein erstes Retentat zu erhalten, das das Zielprotein oder ein anderes Biomolekül umfasst, und ein erstes Permeat, das optional in einem Abfallbehälter oder einem Permeatsammeltank gesammelt wird,

iii) Rückführen und Filtrieren des ersten Retentats, indem das erhaltene erste Retentat in die Tangentialströmungs-Filtrationseinheit zurückgeführt wird, wodurch ein zweites Permeat erhalten wird, das optional gesammelt wird, und ein zweites Retentat erhalten wird, wobei die Rückführung und weitere Filtration in Schritt iii) ein oder mehrere Male ausgeführt wird,

iv) Zugeben einer Elutionsflüssigkeit zu dem zweiten Retentat, um das Zielprotein oder ein anderes Biomolekül aus dem Sorptionsmaterial freizusetzen,

v) Filtrieren des zweiten Retentats unter Verwendung der Tangentialströmungs-Filtrationseinheit, um ein drittes Retentat zu erhalten, das das Sorptionsmaterial umfasst, und ein drittes Permeat, das das Zielprotein oder ein anderes Biomolekül umfasst;

vi) Sammeln des dritten Permeats als eine abgetrennte Produktzusammensetzung, die Protein oder ein anderes Biomolekül umfasst, und wobei ein Druckunterschied zwischen der Retentatseite und der Permeatseite des Filters in der Tangentialströmungs-Filtrationseinheit ausgeglichen oder umgekehrt wird, was dazu führt, dass ein gepulster Druckanstieg und/oder eine Rückspülung des gesammelten ersten Permeats erhalten wird, wobei der Druckunterschied mindestens einmal während des Prozesses während eines oder mehrerer der Schritte ii) bis iv) eingeführt wird, und wobei der gepulste Druckanstieg und/oder die Rückspülung mit einer Frequenz in dem Bereich von 1 - 500 pro Stunde und/oder einer Impulsdauer in dem Bereich von 0,01 bis 200 Sekunden durchgeführt wird.

**Revendications**

1. Appareil comprenant une première entrée (1) et une ou plusieurs premières connexions fluidiques (3) configurées pour acheminer un fluide depuis la première entrée (1) vers une première extrémité (4) d'une unité de filtration à flux tangentiel (5) comprenant un filtre (6) ayant une taille nominale de pore dans la plage de 20 à 300 $\mu$m, de sorte qu'au moins 60 % des particules dont la taille est supérieure à une valeur comprise entre 20 et 300 $\mu$m sont retenues par le filtre ayant une taille moyenne de pore de cette valeur, et dans lequel les premières connexions fluidiques (3) comprennent un matériau de sorption pour une protéine ou autre biomolécule en suspension dans un liquide aqueux à une concentration de 5-70 % en volume, et où une deuxième extrémité (7) de l'unité de filtration à flux tangentiel (5) comporte une deuxième connexion fluidique (8) configurée pour acheminer le rétentat en retour vers la première extrémité de l'unité de filtration à flux tangentiel (5) ; l'unité de filtration à flux tangentiel (5) comportant une troisième connexion fluidique (9) configurée pour acheminer le perméat vers une première sortie (10) ; et dans lequel l'appareil comprend un système de rétrolavage automatique qui est configuré pour appliquer une augmentation de pression et/ou un rétrolavage pulsés du perméat à une fréquence dans la plage de 1-500 par heure et/ou avec une durée d'impulsion comprise entre 0,01 et 200 secondes.

2. Appareil selon la revendication 1, dans lequel le système de rétrolavage automatique comprend :

   - une vanne d'ouverture-fermeture (19) dans la troisième connexion fluidique (9), qui est commandée par une unité de commande électronique (18).

3. Appareil selon l'une quelconque des revendications précédentes, dans lequel le système de rétrolavage automatique comprend :

   - une première vanne de régulation de débit variable (20) dans la deuxième connexion fluidique (8) et une deuxième vanne de régulation de débit variable (21) dans la troisième connexion fluidique (9), qui sont commandées par une unité de commande électronique (18).

4. Appareil selon l'une quelconque des revendications précédentes, dans lequel le système de rétrolavage automatique comprend :

   - une quatrième connexion fluidique (11) configurée pour acheminer le perméat depuis la première sortie (10) vers une cuve de collecte (15) de perméat,
   - une cinquième connexion fluidique (12) reliant la cuve de collecte (15) de perméat au côté perméat du filtre (6), et
   - une pompe centrifuge (16) insérée dans la cinquième connexion fluidique (12), et laquelle pompe centrifuge est également commandée par les unités de commande électroniques (18).

5. Appareil selon l'une quelconque des revendications précédentes, dans lequel une cuve de mélange est raccordée entre la première entrée (1) et les premières connexions fluidiques (3), et dans lequel la cuve de mélange comprend le matériau de sorption.

6. Appareil selon l'une quelconque des revendications précédentes, dans lequel le filtre (6) est un filtre à haut débit présentant un débit de perméat d'eau propre supérieur à 1 000 l/m$^2$/h lorsque la PTM est d'environ 0,1-0,9 bar.

7. Appareil selon l'une quelconque des revendications précédentes, dans lequel le matériau de sorption a une distribution de tailles de particules telle que 90 % en volume des particules ont un diamètre dans la plage de 10 - 1 000 $\mu$m.

8. Appareil selon l'une quelconque des revendications précédentes, dans lequel le matériau de sorption est un matériau de sorption polymère comprenant un ou plusieurs polymères choisis dans le groupe constitué par les polysaccharides naturels, l'agar-agar, l'agarose, le dextrane, la cellulose, les éthers de cellulose, l'hydroxypropylcellulose, la carboxyméthylcellulose, les amidons, une gomme, la gomme de guar, la gomme arabique, la gomme ghatti, la gomme adragante, la gomme de caroube, la gomme xanthane, la pectine, la mucine, la chitine, le chitosane, un alginate, le carraghénane, l'héparine, la gélatine, un polymère synthétique, un polyamide, un polyacrylamide, un polyméthacrylamide, un polyimide, un polyacrylate, un polymère vinylique, le polystyrène, un polyalcène, un alcool polyvinylique, un polyester, un polyéther, un matériau siliceux, le dioxyde de silicium, la silice amorphe, le quartz, la silice, un silicate métallique, un verre à porosité réglée, une céramique, un oxyde métallique, un sulfure, et leurs composites.

**9.** Procédé pour la séparation d'une protéine ou d'une autre biomolécule comprise dans une composition biologique, le procédé comprenant les étapes consistant à :

i) mélanger une composition biologique comprenant une protéine ou autre biomolécule cible avec une composition comprenant un matériau de sorption pour protéine ou autre biomolécule pour obtenir un mélange, la protéine cible ou l'autre biomolécule cible étant portée par le matériau de sorption,

ii) filtrer le mélange en utilisant une unité de filtration à flux tangentiel comportant une membrane ayant une taille nominale de pore dans la plage de 20 à 300 $\mu$m, de sorte qu'au moins 60 % des particules dont la taille est supérieure à une valeur comprise entre 20 et 300 $\mu$m sont retenues par le filtre ayant une taille moyenne de pore de cette valeur, pour obtenir un premier rétentat comprenant la protéine cible ou l'autre biomolécule cible et un premier perméat qui est en option recueilli dans une cuve à déchets ou une cuve de collecte de perméat,

iii) remettre en circulation et filtrer le premier rétentat en renvoyant le premier rétentat obtenu à l'unité de filtration à flux tangentiel et obtenir ainsi un deuxième perméat qui est en option recueilli, et obtenir un deuxième rétentat, et la remise en circulation et la filtration supplémentaire dans l'étape iii) étant effectuées une ou plusieurs fois,

iv) ajouter un liquide d'élution au deuxième rétentat pour libérer la protéine cible ou l'autre biomolécule cible d'avec le matériau de sorption,

v) filtrer le deuxième rétentat en utilisant l'unité de filtration à flux tangentiel pour obtenir un troisième rétentat comprenant le matériau de sorption et un troisième perméat comprenant la protéine cible ou une autre biomolécule cible ;

vi) recueillir le troisième perméat en tant que composition de produit séparée comprenant la protéine ou l'autre biomolécule, et dans lequel une différence de pression entre le côté rétentat et le côté perméat du filtre dans l'unité de filtration à flux tangentiel est égalisée ou inversée, ce qui conduit à obtenir une augmentation de pression et/ou un rétrolavage pulsés du premier perméat recueilli, ladite différence de pression étant introduite au moins une fois au cours du procédé pendant une ou plusieurs des étapes ii) à iv), et dans lequel l'augmentation de pression et/ou le rétrolavage pulsés sont effectués a une fréquence dans la plage de 1-500 par heure et/ou avec une durée d'impulsion dans la plage de 0,01 à 200 secondes.

Fig. 1

Fig. 2

Fig. 3

Fig. 4

EP 4 408 572 B1

Fig. 5

36

Fig. 6

Fig. 7

Fig. 8

Fig. 9

Fig. 10

← beta-lg

1 2 3 4 5 6 7

Fig. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015118223 A1
- US 6022477 A

- WO 2015118223 A